# EUROPEAN PATENT SPECIFICATION

(11) **EP 1 932 541 B1**
(45) Date of publication and mention of the grant of the patent: **18.12.2013**
(21) Application number: 06782682.6
(22) Date of filing: 04.08.2006
(51) Int. Cl.: A61K 45/00, A61K 31/7088, A61K 48/00, A61P 35/00, A61P 43/00, G01N 33/15, G01N 33/50

(54) **APOPTOSIS PROMOTER, CELL PROLIFERATION INHIBITOR, PROPHYLACTIC/THERAPEUTIC AGENT FOR CANCER, SCREENING METHOD FOR THE PROMOTER, INHIBITOR OR AGENT**
APOPTOSE-PROMOTOR, ZELLPROLIFERATIONSHEMMER, PROPHYLAKTISCHES MITTEL GEGEN KREBS, SCREENING-VERFAHREN FÜR DEN PROMOTOR, HEMMER ODER DAS MITTEL
PROMOTEUR DE L'APOPTOSE, INHIBITEUR DE LA PROLIFERATION CELLULAIRE, AGENT PROPHYLACTIQUE/THERAPEUTIQUE POUR LE CANCER, PROCEDE DE CRIBLAGE POUR LE PROMOTEUR, INHIBITEUR OU AGENT

(30) Priority: 04.08.2005 JP 2005227274
(43) Date of publication of application: 18.06.2008
(73) Proprietor: OSAKA UNIVERSITY, Suita-shi, Osaka 565-0871 (JP)
(72) Inventor: TSUJIKAWA, Kazutake, Suita-shi, Osaka 565-0871 (JP); YAMAMOTO, Hiroshi, Suita-shi, Osaka 565-0871 (JP); KONISHI, Noboru, Kashihara-shi, Nara 634-8521 (JP)
(74) Representative: Duckworth, Timothy John
(86) International application number: PCT/JP2006/315896
(87) International publication number: WO 2007/015587

(56) References cited:
- WO-A1-2005/007849
- WO-A1-2005/080564
- WO-A2-2004/076613
- TSUJIKAWA KAZUTAKE; KOIKE KAZUO; SHINKAWA AINA; ARIMA HIROSHI; KITAE KAORI; IWAO MUTSURM; YAMAMOTO HIROSHI; SHIMADA KEIJI; KONISHI: "Identification and expression of the oxidative demethylase PCA-1 in prostate cancer" FASEB JOURNAL, FED. OF AMERICAN SOC. FOR EXPERIMENTAL BIOLOGY, BETHESDA, MD, US, vol. 20, no. 5, Part 2, 5 April 2006 (2006-04-05), page A1360, XP009121971 ISSN: 0892-6638
- KONISHI N. ET AL.: 'High expression of a new marker PCA-1 in human prostate carcinoma' CLIN. CANCER RES. vol. 11, no. 14, 15 July 2005, pages 5090 - 5097, XP003003791
- KATAOKA T. ET AL.: 'The caspase-8 inhibitor FLIP promotes activation of NF-kappaB and Erk signaling pathways' CURR. BIOL. vol. 10, no. 11, 2000, pages 640 - 648, XP003003792

### SEQUENCE LISTING

<110> Osaka University PCAInterMed, Inc. Link Genomics, Inc.
<120> Apoptosis promoting agent, cell growth inhibiting agent, prophylactic or therapeutic agent for tumor, and screening method therefor
<130> 09954
<150> JP 2005-227274
   <151> 2005-08-04
<160> 11
<170> PatentIn version 3.2
<210> 1
   <211> 1520
   <212> DNA
   <213> Homo sapiens
<220>
   <221> CDS
   <222> (407).. (1267)
<400> 1
   aggtcacaga ctgcggagtg ggtcaggggc tgcgagggct gccccaagtc ctaccgggtt 60
   tgcacgggcg cgcccggctc cgcccgcaag tgcgccttcc tgacttactg ctgggtgcgc 120
   ggggctgggg gtgcgagtac cacccctgaa gtctcttcct gggcgacctc cggggcctca 180
   ttctaggcct ccttaaagag aaggatctaa attaggaaaa ggaagtgccc ttatccacga 240
   ccaagctctt ccacctgcgg agctcgctta gtctgcacct caaccgtgcg gaaagtgact 300
   gccctgttta ctgaggaaaa actggggctc agaaagatac catggagtag tttgaaacag 360
   gaacaaaatc ttctgaaagc tcggagcaga agcctttttg gtcaac atg gag gaa       415
                                                      Met Glu Glu
                                                      1
   aaa aga cgg cga gcc cga gtt cag gga gcc tgg gct gcc cct gtt aaa    463
   Lys Arg Arg Arg Ala Arg Val Gln Gly Ala Trp Ala Ala Pro Val Lys
       5                   10                  15
   agc cag gcc att gct cag cca gct acc act gct aag agc cat ctc cac    511
   Ser Gln Ala Ile Ala Gln Pro Ala Thr Thr Ala Lys Ser His Leu His
   20                  25                  30                  35
   cag aag cct ggc cag acc tgg aag aac aaa gag cat cat ctc tct gac    559
   Gln Lys Pro Gly Gln Thr Trp Lys Asn Lys Glu His His Leu Ser Asp
                   40                  45                  50
   aga gag ttt gtg ttc aaa gaa cct cag cag gta gta cgt aga gct cct    607
   Arg Glu Phe Val Phe Lys Glu Pro Gln Gln Val Val Arg Arg Ala Pro
               55                  60                  65
   gag cca cga gtg att gac aga gag ggt gtg tat gaa atc agc ctg tca    655
   Glu Pro Arg Val Ile Asp Arg Glu Gly Val Tyr Glu Ile Ser Leu Ser
           70                  75                  80
   ccc aca ggt gta tct agg gtc tgt ttg tat cct ggc ttt gtt gac gtg    703
   Pro Thr Gly Val Ser Arg Val Cys Leu Tyr Pro Gly Phe Val Asp Val
       85                  90                  95
   aaa gaa gct gac tgg ata ttg gaa cag ctt tgt caa gat gtt ccc tgg    751
   Lys Glu Ala Asp Trp Ile Leu Glu Gln Leu Cys Gln Asp Val Pro Trp
   100                 105                 110                 115
   aaa cag agg acc ggc atc aga gag gat ata act tat cag caa cca aga    799
   Lys Gln Arg Thr Gly Ile Arg Glu Asp Ile Thr Tyr Gln Gln Pro Arg
                   120                 125                 130
   ctt aca gca tgg tat gga gaa ctt cct tac act tat tca aga atc act    847
   Leu Thr Ala Trp Tyr Gly Glu Leu Pro Tyr Thr Tyr Ser Arg Ile Thr
               135                 140                 145
   atg gaa cca aat cct cac tgg cac cct gtg ctg cgc aca cta aag aac      895
   Met Glu Pro Asn Pro His Trp His Pro Val Leu Arg Thr Leu Lys Asn
           150                 155                 160
   cgc att gaa gag aac act ggc cac acc ttc aac tcc tta ctc tgc aat      943
   Arg Ile Glu Glu Asn Thr Gly His Thr Phe Asn Ser Leu Leu Cys Asn
       165                 170                 175
   ctt tat cgc aat gag aag gac agc gtg gac tgg cac agt gat gat gaa      991
   Leu Tyr Arg Asn Glu Lys Asp Ser Val Asp Trp His Ser Asp Asp Glu
   180                 185                 190                 195
   ccc tca cta ggg agg tgc ccc att att gct tca cta agt ttt ggt gcc     1039
   Pro Ser Leu Gly Arg Cys Pro Ile Ile Ala Ser Leu Ser Phe Gly Ala
                   200                 205                 210
   aca cgc aca ttt gag atg aga aag aag cca cca cca gaa gag aat gga     1087
   Thr Arg Thr Phe Glu Met Arg Lys Lys Pro Pro Pro Glu Glu Asn Gly
               215                 220                 225
   gac tac aca tat gtg gaa aga gtg aag ata ccc ttg gat cat ggg acc     1135
   Asp Tyr Thr Tyr Val Glu Arg Val Lys Ile Pro Leu Asp His Gly Thr
           230                 235                 240
   ttg tta atc atg gaa gga gcg aca caa gct gac tgg cag cat cga gtg     1183
   Leu Leu Ile Met Glu Gly Ala Thr Gln Ala Asp Trp Gln His Arg Val
       245                 250                 255
   ccc aaa gaa tac cac tct aga gaa ccg aga gtg aac ctg acc ttt cgg     1231
   Pro Lys Glu Tyr His Ser Arg Glu Pro Arg Val Asn Leu Thr Phe Arg
   260                 265                 270                 275
   aca gtc tat cca gac cct cga ggg gca ccc tgg tga cgtcagagct          1277
   Thr Val Tyr Pro Asp Pro Arg Gly Ala Pro Trp
                   280                 285
   ttgagagaga agcttcactg aaacggagca aaccttccac tgagaagcca cttcaagagg   1337
   ctggtgctgc tagatctcat gatgtggctg ttgggaagat ggtggggttt gtttgccagc   1397
   ttggagtcct attaaatgaa agccagcaac tcatgttggt aataggtcta ctgtgggaac   1457
   agttatccct aaccacagct caaaatcgct atcatcttta ggcaaattaa aatctatgtg 1517
   gca 1520
<210> 2
   <211> 286
   <212> PRT
   <213> Homo sapiens
<400> 2
   Met Glu Glu Lys Arg Arg Arg Ala Arg Val Gln Gly Ala Trp Ala Ala
   1               5                   10                  15
   Pro Val Lys Ser Gln Ala Ile Ala Gln Pro Ala Thr Thr Ala Lys Ser
               20                  25                  30
   His Leu His Gln Lys Pro Gly Gln Thr Trp Lys Asn Lys Glu His His
           35                  40                  45
   Leu Ser Asp Arg Glu Phe Val Phe Lys Glu Pro Gln Gln Val Val Arg
       50                  55                  60
   Arg Ala Pro Glu Pro Arg Val Ile Asp Arg Glu Gly Val Tyr Glu Ile
   65                  70                  75                  80
   Ser Leu Ser Pro Thr Gly Val Ser Arg Val Cys Leu Tyr Pro Gly Phe
                   85                  90                  95
   Val Asp Val Lys Glu Ala Asp Trp Ile Leu Glu Gln Leu Cys Gln Asp
               100                 105                 110
   Val Pro Trp Lys Gln Arg Thr Gly Ile Arg Glu Asp Ile Thr Tyr Gln
   115 120 125
   Gln Pro Arg Leu Thr Ala Trp Tyr Gly Glu Leu Pro Tyr Thr Tyr Ser
   130 135 140
   Arg Ile Thr Met Glu Pro Asn Pro His Trp His Pro Val Leu Arg Thr
   145 150 155 160
   Leu Lys Asn Arg Ile Glu Glu Asn Thr Gly His Thr Phe Asn Ser Leu
   165 170 175
   Leu Cys Asn Leu Tyr Arg Asn Glu Lys Asp Ser Val Asp Trp His Ser
   180 185 190
   Asp Asp Glu Pro Ser Leu Gly Arg Cys Pro Ile Ile Ala Ser Leu Ser
   195 200 205
   Phe Gly Ala Thr Arg Thr Phe Glu Met Arg Lys Lys Pro Pro Pro Glu
   210 215 220
   Glu Asn Gly Asp Tyr Thr Tyr Val Glu Arg Val Lys Ile Pro Leu Asp
   225 230 235 240
   His Gly Thr Leu Leu Ile Met Glu Gly Ala Thr Gln Ala Asp Trp Gln
   245 250 255
   His Arg Val Pro Lys Glu Tyr His Ser Arg Glu Pro Arg Val Asn Leu
   260 265 270
   Thr Phe Arg Thr Val Tyr Pro Asp Pro Arg Gly Ala Pro Trp
   275 280 285
<210> 3
   <211> 18
   <212> RNA
   <213> Artificial
<220>
   <223> siRNA against human PCA-1
<400> 3
   ccgagaguga accugacc 18
<210> 4
   <211> 21
   <212> RNA
   <213> Artificial
<220>
   <223> siRNA to PCA-1
<400> 4
   gaaagaagcu gacuggauau u 21
<210> 5
   <211> 21
   <212> RNA
   <213> Artificial
<220>
   <223> siRNA to PCA-1
<400> 5
   gcacauuuga gaugagaaau u 21
<210> 6
   <211> 21
   <212> RNA
   <213> Artificial
<220>
   <223> siRNA to PCA-1
<400> 6
   gagagaagcu ucacugaaau u 21
<210> 7
   <211> 19
   <212> RNA
   <213> Artificial
<220>
   <223> control siRNA
<400> 7
   auccgcgcga uaguacgua 19
<210> 8
   <211> 23
   <212> DNA
   <213> Artificial
<220>
   <223> primer
<400> 8
   taccactgct aagagccatc tcc 23
<210> 9
   <211> 23
   <212> DNA
   <213> Artificial
<220>
   <223> primer
<400> 9
   acctgctgag gttctttgaa cac 23
<210> 10
   <211> 20
   <212> DNA
   <213> Artificial
<220>
   <223> primer
<400> 10
   ccatcaccat cttccaggag 20
<210> 11
   <211> 19
   <212> DNA
   <213> Artificial
<220>
   <223> primer
<400> 11
   aatgagccca gccttctcc 19

## Description

### Technical Field

The present invention relates to an apoptosis promoting agent, a cell growth inhibitor, or a prophylactic/therapeutic agent for cancer, comprising a compound that suppresses the expression or function of PCA-1 as an active ingredient. The present invention also relates to a screening method for a compound for promoting apoptosis, a compound for inhibiting cell growth, or a compound for preventing/treating cancer, comprising selecting a compound capable of suppressing the expression or function of PCA-1.

### Background Art

Prostate cancer is a malignant tumor ranking high among male cancers in terms of morbidity and mortality in Europe and US. In Japan, the morbidity and mortality of prostate cancer have recently been rising rapidly with the westernization of dietary life and general population aging.

In order to search for a target molecule for the treatment of prostate cancer, the present inventors histopathologically divided prostate tissue isolated from a prostate cancer patient into a tumor portion and a non-tumor portion, and analyzed genes showing differential expression between the non-tumor portion and the tumor portion. As a result, the present inventors succeeded in cloning a gene called PCA-1 (also referred to as human AlkB homologue 3 (hABH3)) as a gene expressed at higher levels specifically in the tumor portion than in the non-tumor portion (Proceedings of the 123rd Annual Meeting of the Pharmaceutical Society of Japan No.4, p.15, 2003). Recently, PCA-1 was identified as an enzyme for repair of DNA and RNA alkylation damage, and was suggested to be relevant to cancer prophylaxis (Nature, vol.421, p859-863, 2003/Proc. Natl. Acad. Sci. USA, vol.99, No.26, pp.16660-16665, 2002). The present inventors found that the survival rate for a prostate cancer cell line treated with the alkylating agent methyl methanesulfonate increased with the introduction of a PCA-1 expression vector (Program and Proceedings of the 26th Annual Meeting of the Molecular Biology Society of Japan, p.525, 2003).

However, the biological functions of PCA-1 in prostate cancer cell are unclear. Hence, there is a demand for elucidating the biological functions of PCA-1 in prostate cancer cell, and developing a prophylactic/therapeutic drug for prostate cancer based thereon.

It is an object of the present invention to elucidate the biological functions of PCA-1 in prostate cancer cells, and to provide a cancer prophylactic/therapeutic drug, a screening method therefor and the like based on the function.

### Disclosure of the Invention

With the aim of accomplishing the above-described objects, the present inventors diligently analyzed the functions of PCA-1 on the growth and anticancer agent susceptibility of prostate cancer cells. As a result, when a prostate cancer cell line was allowed to highly express PCA-1, an increase in the expression of FLICE-like inhibitory protein (FLIP) and FLIP-dependent promotion of the Raf-1/extracellular stress-regulated kinase (ERK) signal were confirmed; the growth potential of cancer cells via growth factors such as EGF was significantly enhanced. PCA-1 was also found to inhibit the tumor necrosis factor-related apoptosis-inducing ligand (TRAIL) via FLIP, and to inhibit paclitaxel-induced apoptosis via the Raf-1/ERK/signal. Meanwhile, because the same phenomenon was not observed when the expression of endogenous PCA-1 was knocked down with siRNA, even when FLIP was highly expressed, it was thought that PCA-1 is essential for the functioning of the FLIP/Raf-1/ERK signal. From these findings, the present inventors found that PCA-1 plays an important role as a factor for the mechanism for the acquirement of anticancer-agent resistance and cell growth in prostate cancer cells, and that by suppressing the expression or function of PCA-1, it is possible to inhibit anticancer-agent resistance, to suppress cell growth, and to prevent/treat prostate cancer, and developed the present invention. Accordingly, the present invention relates to the following:
[1] A compound for use in preventing or treating cancer by promoting apoptosis which is induced by FASL, TRAIL or TNF-α, wherein the compound is (i) or (ii) below:
   (i) a compound that suppresses the expression of PCA-1 that is a nucleic acid having a nucleotide sequence or a portion thereof complementary to the nucleotide sequence that encodes the PCA-1 polypeptide;
   (ii) a compound that suppresses the function of PCA-1 that is an antibody that specifically recognizes the PCA-1 polypeptide or a nucleic acid having the nucleotide sequence that encodes the same.
[2] A compound for use in preventing or treating cancer by promoting apoptosis which is induced by paclitaxel, wherein the compound is (i) or (ii) below:
   (i) a compound that suppresses the expression of PCA-1 that is a nucleic acid having a nucleotide sequence or a portion thereof complementary to the nucleotide sequence that encodes the PCA-1 polypeptide;
   (ii) a compound that suppresses the function of PCA-1 that is an antibody that specifically recognizes the PCA-1 polypeptide or a nucleic acid having the nucleotide sequence that encodes the same.
[3] A compound for use in the prophylactic/therapeutic treatment of a prostate cancer, wherein the compound is (i) or (ii) below;
   (i) a compound that suppresses the expression of PCA-1 that is a nucleic acid having a nucleotide sequence or a portion thereof complementary to the nucleotide sequence that encodes the PCA-1 polypeptide;
   (ii) a compound that suppresses the function of PCA-1 that is an antibody that specifically recognizes the PCA-1 polypeptide or a nucleic acid having the nucleotide sequence that encodes the same.
[4] An *in vitro* screening method for a compound for promoting the ubiquitination of FLIP, comprising the following steps:
   (1) a step for treating PCA-1 expressing cells with a test compound in the presence of proteasome inhibitor;
   (2) a step for evaluating the ubiquitination of FLIP in said cells.
[5] An *in vitro* screening method for a compound for preventing or treating prostate cancer, comprising the following steps:
   (1) a step for treating PCA-1-expressing cells with paclitaxel in the presence of a test compound;
   (2) a step for evaluating the degree of the apoptosis of said cells.
[6] The method described in [4] wherein a compound for preventing/treating cancer is identified.
[7] The method of [6] wherein the cancer is prostate cancer.

Because a compound that suppresses the expression or function of PCA-1, which is an active ingredient of the agent of the present invention, cancels the apoptosis inhibitory action and cell growth enhancing action of PCA-1, and reduces the anticancer-agent resistance and cell growth of cancer cells, the compound is useful as an apoptosis promoting agent, a cell growth inhibitor, and a prophylactic/therapeutic agent for cancer. By selecting a compound capable of suppressing the expression or function of PCA-1, a compound for promoting apoptosis, a compound for inhibiting cell growth, and a compound for preventing or treating cancer, having a novel action mechanism, can be screened for.

### Brief Description of the Drawings

FIG. 1 shows the results of Western blotting showing increased expression of PCA-1 protein in two clones (Clone 2, Clone 31) of a PCA-1-overexpressing cell line (DU145/PCA-1). HygB indicates a control incorporating an empty vector.
FIG. 2 is a graph showing cell growth (BrdU uptake) in the PCA-1-overexpressing cell line. White column: HygB, shaded column: Clone 2, black column: Clone 31 (the same applies to FIGS. 3, 9, and 11).
FIG. 3 is a graph showing the effect of an ERK inhibitor (U0126) on the cell growth of the PCA-1-overexpressing cell line in the presence of EGF.
FIG. 4 shows the results of Western blotting showing the activation of EGF-induced ERK and the expression of Cyclin D1 in the PCA-1-overexpressing cell line, and the effects of an ERK inhibitor (U0126) thereon. pERK: phospho-ERK.
FIG. 5 shows the results of Western blotting showing increased expression of FLIP protein in the PCA-1-overexpressing cell line.
FIG. 6 shows the results of Western blotting showing the inhibition of the ubiquitination of FLIP protein in the PCA-1-overexpressing cell line. I.B.: immunoblotting, *: ubiquitinated FLIP.
FIG. 7 shows the results of Western blotting showing increased expression of FLIP protein in two clones (Clone 1, Clone 12) of an FLIP-overexpressing cell line (DU145/FLIP).
FIG. 8 shows the results of Western blotting showing the FLIP-Raf-1 interaction in the FLIP-overexpressing cell line, and the effect of PCA-1 siRNA thereon. I.P.: immunoprecipitation.
FIG. 9 shows the inhibition of paclitaxel-induced apoptosis by PCA-1 (upper graph) and the induction of the pMEK1-Raf-1 interaction (lower panel).
FIG. 10 shows the actions of U0126 and PD98059 on the paclitaxel-induced apoptosis inhibitory effect and ERK activating effect of PCA-1.
FIG. 11 shows the inhibition of TRAIL-induced apoptosis by PCA-1.
FIG. 12 schematically shows the effects of PCA-1 on cell growth and apoptosis.

### Best Mode for Carrying out the Invention

### (1. A compound that suppresses the expression or function of PCA-1)

As shown in an Example below and the like, when PCA-1 is highly expressed, paclitaxel- or TRAIL-induced apoptosis is inhibited, cell growth via MAP kinase signaling such as by EGF is accentuated, and anticancer-agent resistance is enhanced. From this fact, a compound that suppresses the expression or function of PCA-1 is understood to be capable of inhibiting the anticancer agent resistance of cancer cells and preventing/treating cancer by promoting apoptosis induced by paclitaxel, TRAIL and the like, and inhibiting cell growth via MAP kinase signaling such as by EGF. Therefore, an agent comprising a compound that suppresses the expression or function of PCA-1 as an active ingredient is useful in promoting apoptosis, inhibiting cell growth, and preventing/ treating cancer.

The expression of PCA-1 refers to a state wherein the translation product of PCA-1 (i.e., polypeptide) is produced and localized to the action site thereof in a functional manner.

A function of PCA-1 refers to a biological function (activity) exhibited by the translation product of PCA-1. As shown in an Example below, when PCA-1 is highly expressed, the ubiquitination of FLIP is inhibited, the amount of FLIP polypeptide expressed increases, the FLIP-Raf-1 interaction increases, MAP kinase signaling is enhanced, and cyclin D1 expression increases. Therefore, as examples of the function of PCA-1, a function to inhibit the ubiquitination of FLIP (including a function to hence increase the amount of FLIP polypeptide expressed), a function to increase the FLIP-Raf-1 interaction, a function to enhance MAP kinase signaling, a function to increase cyclin D1 expression and the like can be mentioned. In addition, a function to repair DNA and RNA alkylation damage (Nature, (UK), vol. 421, p859-863, 2003) and the like are also included in the functions of PCA-1.

Ubiquitination refers to the phenomenon in which many ubiquitin units bind to protein in a chain form. A ubiquitinated protein is recognized by proteasome and degraded. Therefore, if the ubiquitination of FLIP is inhibited by PCA-1, the FLIP degradation rate will decrease, resulting in an increase in the amount of FLIP polypeptide expressed in the cells.

FLIP (FADD-like ICE inhibitory protein) is known to act as a dominant negative molecule for caspase 8 (Yonehara S., Cell Struct. Funct., 28(1), 1-2, 2003). That is, caspase 8 interacts with FADD to induce caspase 8/FADD-dependent apoptosis such as FASL-induced (via FAS) apoptosis, TRAIL-induced (via DR4 or DR5) apoptosis, and TNF-α-induced (via TNFR) apoptosis, whereas FLIP suppresses caspase 8/FADD-dependent apoptosis by binding to FADD in place of caspase 8. FLIP is known to interact with Raf-1 to activate Raf-1 (Kataoka T., et al, Curr. Biol., 10(11), 640-648, 2000). Therefore, as the amount of FLIP polypeptide expressed in the cells increases, caspase 8/FADD-dependent apoptosis is suppressed, the FLIP-Raf-1 interaction increases, and Raf-1 is activated.

The activated Raf-1 activates the phosphorylation cascade of Raf-1/MEK1/ERK (MAP kinase signaling). Upon activation of MAP kinase signaling, various transcription factors that are present downstream thereof are directly or indirectly activated, the expression of a variety of genes involved in cell growth, differentiation and the like (cyclin D1 and the like) is induced, and cell growth and differentiation are induced.

In the the present invention, the compound that suppresses the expression of PCA-1 may act in any one of the stages of PCA-1 transcription, post-transcriptional regulation, translation, post-translational modification, localization, protein folding and the like.

A compound that suppresses the function of PCA-1 refers to a compound having an action to suppress one of the above-described functions of PCA-1 (for example, a function to inhibit the ubiquitination of FLIP, a function to increase the FLIP-Raf-1 interaction, a function to enhance MAP kinase signaling, a function to increase cyclin D1 expression and the like) by binding to the PCA-1 polypeptide, by modifying the polypeptide, or by reducing the stability of the polypeptide, and the like.

As examples of the compound that suppresses the expression or function of PCA-1, (i) and (ii) below and the like can be mentioned.
(i) A nucleic acid having a nucleotide sequence or a portion thereof complementary to the nucleotide sequence that encodes the PCA-1 polypeptide;
(ii) an antibody that specifically recognizes the PCA-1 polypeptide, a dominant negative mutant of the PCA-1 polypeptide, or a nucleic acid having the nucleotide sequence that encodes the same.

In the present invention, the PCA-1 polypeptide can be the PCA-1 polypeptide of an optionally chosen mammal. As the mammal, human and non-human mammals can be mentioned. As examples of non-human mammals, laboratory animals such as mice, rats, hamsters, guinea pigs, and other rodents, as well as rabbits, domestic animals such as swine, bovine, goat, horses, and sheep, companion animals such as dogs and cats, and primates such as monkeys, orangutans, and chimpanzees can be mentioned. The mammal is preferably a human.

The PCA-1 polypeptide of a mammal is preferably a wild-type polypeptide; for example, a human wild-type PCA-1 polypeptide (for example, a polypeptide consisting of the amino acid sequence shown by SEQ ID NO:2) and the like can be mentioned.

Polypeptides consisting of substantially the same amino acid sequence as a wild-type PCA-1 polypeptide are also included in the "PCA-1 polypeptide". As "a protein having substantially the same amino acid sequence", polypeptides consisting of an amino acid sequence having a homology of about 90% or more, preferably 95% or more, more preferably about 98% or more, to the amino acid sequence of the wild-type PCA-1 polypeptide, and having substantially the same quality of function as the wild-type PCA-1 polypeptide and the like can be mentioned.

Here, "a homology" means the proportion (%) of identical amino acid residues and analogous amino acid residues to all overlapping amino acid residues in the optimal alignment where two amino acid sequences are aligned using a mathematical algorithm known in the technical field (preferably, the algorithm is such that a gap can be introduced into one or both of the sequences for the optimal alignment). "An analogous amino acid" means an amino acid having similar physiochemical properties; examples thereof include amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr) and amino acids having a small side-chain (Gly, Ala, Ser, Thr, Met). Substitution by such analogous amino acids is expected not to change the phenotype of a polypeptide (i.e., conservative amino acid substitution). Specific examples of the conservative amino acid substitution are well known in the technical field and are described in various documents (see, for example, Bowie et al., Science, 247:1306-1310 (1990)).

Algorithms to determine amino acid sequence homology include, for example, but are not limited to, the algorithm described in Karlin et al., Proc. Natl. Acad. Sci. USA, 90:5873-5877 (1993) [the algorithm is incorporated in the NBLAST and XBLAST programs (version 2.0) (Altschul et al., Nucleic Acids Res., 25:3389-3402 (1997))], the algorithm described in Needleman et al., J. Mol. Biol., 48:444-453 (1970) [the algorithm is incorporated in the GAP program in the GCG software package], the algorithm described in Myers and Miller, CABIOS, 4:11-17 (1988) [the algorithm is incorporated in the ALIGN program (version 2.0), which is part of the CGC sequence alignment software package], the algorithm described in Pearson et al., Proc. Natl. Acad. Sci. USA, 85:2444-2448 (1988) [the algorithm is incorporated in the FASTA program in the GCG software package] and the like. Amino acid sequence homology can be calculated as appropriate with the above-described program using default parameters thereof. For example, amino acid sequence homology can be calculated using the homology calculation algorithm NCBI BLAST-2 (National Center for Biotechnology Information Basic Local Alignment Search Tool) under the following conditions (matrix=BLOSUM62; gap open=11; gap extension=1; x_ dropoff=50; expectancy=10; filtering=ON).

"Substantially the same quality of function" means that, for example, the function of the PCA-1 polypeptide is qualitatively equivalent to the function of the wild-type PCA-1 polypeptide. Therefore, it is preferable that the function of the PCA-1 polypeptide be equivalent to the function of the wild-type PCA-1 polypeptide (for example, about 0.1 to 10 times, preferably about 0.5 to 2 times), but the quantitative factors, such as the extent of activity and the molecular weight of the protein, may be different. As mentioned herein, the function of the PCA-1 polypeptide includes the above-described functions (a function to inhibit the ubiquitination of FLIP, a function to increase the FLIP-Raf-1 interaction, a function to enhance MAP kinase signaling, a function to increase cyclin D expression, a function to repair DNA and RNA alkylation damage) and the like. The individual functions can be evaluated by the methods described in the section below (2. A screening method comprising selecting a compound capable of suppressing the expression or function of PCA-1).

As the nucleotide sequence that encodes the PCA-1 polypeptide, the nucleotide sequences of cDNA, mRNA, initial transcription product (immature mRNA), and chromosome DNA that encode the PCA-1 polypeptide are included; more specifically, for example, a nucleotide sequence of a cDNA that encodes the human wild-type PCA-1 polypeptide (for example, SEQ ID NO:1), a nucleotide sequence of a chromosome DNA that encodes the human wild-type PCA-1 polypeptide (for example, GenBank accession number: NT_009237) and the like can be mentioned.

A nucleic acid having a nucleotide sequence that is complementary to the target region of the desired nucleic acid, that is, a nucleic acid capable of hybridizing to the desired nucleic acid under physiological conditions (for example, intracellular and the like) can be said to be "antisense" against the desired nucleic acid. As used herein, "complementary" refers to having a complementarity between nucleotide sequences of about 70% or more, preferably about 80% or more, more preferably about 90% or more, still more preferably about 95% or more, most preferably 100%. Nucleotide sequence homology in the present description can be calculated using the homology calculation algorithm NCBI BLAST (National Center for Biotechnology Information Basic Local Alignment Search Tool) under the following conditions (expectancy=10; gap allowed; filtering=ON; match score=1; mismatch score=-3). As preferable examples of other algorithms for determining nucleotide sequence homology, the above-described amino acid sequence homology calculation algorithms can be mentioned.

A nucleic acid having a nucleotide sequence or a portion thereof complementary to the nucleotide sequence that encodes the PCA-1 polypeptide (hereinafter also referred to as "antisense PCA-1") can be designed and synthesized on the basis of information on the nucleotide sequence that encodes a cloned or determined PCA-1 polypeptide. Such a nucleic acid is capable of inhibiting the replication or expression of the PCA-1 gene. Hence, antisense PCA-1 is capable of hybridizing to an RNA (mRNA or initial transcription product) transcribed from the gene (chromosome DNA) that encodes the PCA-1 polypeptide under physiological conditions (for example, intracellular and the like) to inhibit the synthesis (processing) or function (translation into protein) of the mRNA.

The target region of antisense PCA-1 is not limited with respect to length, as long as the translation into PCA-1 polypeptide is inhibited as a result of hybridization of antisense nucleic acid; the target region may be the entire sequence or a partial sequence of the mRNA or initial transcription product that encodes the polypeptide, and the length is about 15 bases for the shortest, and the entire sequence of the mRNA or initial transcription product for the longest. Considering the ease of synthesis, oligonucleotides consisting of about 15 to about 30 bases are preferable, but these are not limiting. Specifically, for example, the 5' end hairpin loop; 5' end 6-base-pair repeats, 5' end untranslated region, translation initiation codon, protein coding region, translation termination codon, 3' end untranslated region, 3' end palindrome region, and 3' end hairpin loop of a nucleic acid that encodes PCA-1 (for example, mRNA or initial transcription product) may be selected as target regions, but any other region in the nucleic acid that encodes PCA-1 may be selected as the target. For example, the intron portion of the PCA-1 gene is also the preferable target region.

Furthermore, antisense PCA-1 may be one not only capable of hybridizing to the mRNA or initial transcription product that encodes the PCA-1 polypeptide to inhibit its translation into a polypeptide, but also capable of binding to the double-stranded DNA that encodes the PCA-1 polypeptide to form a triple strand (triplex) to inhibit the transcription of the RNA.

The kind of the antisense nucleic acid may be DNA or RNA, or a DNA/RNA chimera. Because a natural form antisense nucleic acid easily undergoes degradation of the phosphor-diester bond thereof by a nuclease present in the cells, an antisense nucleic acid can also be synthesized using a modified nucleotide of the thiophosphate form (P=O in phosphate bond replaced with P=S), 2'-O-methyl form and the like, which are stable to decomposing enzymes. Other important factors for the designing of antisense nucleic acids include increases in water-solubility and cell membrane permeability and the like; these can also be cleared by choosing appropriate dosage forms such as those using liposome or microspheres.

A ribozyme capable of specifically cleaving the mRNA or initial transcription product that encodes PCA-1 in the coding region (including the intron portion in the case of initial transcription product) can also be included in antisense PCA-1. "Ribozyme" refers to an RNA having an enzyme activity to cleave a nucleic acid; since it has recently been found that an oligo-DNA having the nucleotide sequence of the enzyme activity site also has the same nucleic acid cleavage activity, this term is herein used as a concept including DNA, as long as it possesses sequence-specific nucleic acid cleavage activity. The most versatile ribozyme is self-splicing RNA, found in infectious RNAs such as viroid and virosoid, and is known to occur in the hammerhead type, the hairpin type and the like. The hammerhead type exhibits enzyme activity with about 40 bases, and it is possible to specifically cleave only the target mRNA by rendering several bases (about 10 bases in total) at each end adjacent to the hammerhead structure moiety a sequence complementary to the desired cleavage site of the mRNA. Furthermore, when a ribozyme is used in the form of an expression vector comprising the DNA that encodes the same, a hybrid ribozyme wherein a sequence modified from tRNA is further joined to promote the localization of the transcription product to cytoplasm may be used [Nucleic Acids Res., 29(13): 2780-2788 (2001)].

A double stranded oligo RNA (siRNA) having a nucleotide sequence complementary to a partial sequence in the coding region of the mRNA or initial transcription product that encodes PCA-1 (including the intron portion in the case of initial transcription product) is also included in antisense PCA-1. It had been known that so-called RNA interference (RNAi), which is a phenomenon that if short double stranded RNA is introduced into cells, an mRNA complementary to one strand of the RNA is degraded, occurs in nematodes, insects, plants and the like; since this phenomenon was confirmed to also occur in mammalian cells [Nature, 411(6836): 494-498 (2001)], siRNA has been widely utilized as an alternative technique to ribozymes. The size of siRNA is not limited, as long as RNAi can be induced; for example, the size can be not less than 15 bp, preferably not less than 20 bp. An siRNA having RNAi activity can be prepared by synthesizing a sense strand and an antisense strand using an automated DNA/RNA synthesizer, denaturing them in an appropriate annealing buffer solution, for example, at about 90 to about 95°C for about 1 minute, and then annealing them at about 30 to about 70°C for about 1 to about 8 hours.

An expression vector capable of expressing the above-described nucleic acid having a nucleotide sequence or a portion thereof complementary to the nucleotide sequence that encodes the PCA-1 polypeptide is also preferable as a compound that suppresses the expression or function of PCA-1. The expression vector is preferably an expression vector capable of functioning in cells (prostate cancer cells and the like) of the subject mammal, and can be provided in a manner wherein a nucleic acid having a nucleotide sequence or a portion thereof complementary to the nucleotide sequence that encodes the PCA-1 polypeptide is functionally joined downstream of an appropriate promoter (for example, promoters capable of exhibiting promoter activity in cells (prostate cancer cells and the like) of the subject mammal) in the vector.

As the expression vector, plasmid vectors, viral vectors and the like can be mentioned; vectors suitable for use in cells (prostate cancer cells and the like) of mammals such as humans include viral vectors such as adenovirus, retrovirus, adeno-associated virus, herpesvirus, vaccinia virus, poxvirus, poliovirus, Sindbis virus, Sendai virus, and Epstein-Barr virus.

Any promoter can be used, as long as it is capable of exhibiting promoter activity in cells (prostate cancer cells and the like) of the subject mammal; examples include viral promoters such as SV40-derived initial promoter, cytomegalovirus LTR, Rous sarcoma virus LTR, MoMuLV-derived LTR, and adenovirus-derived initial promoter, and mammalian constitutive protein gene promoters such as β-actin gene promoter, PGK gene promoter, and transferrin gene promoter and the like.

The expression vector can contain a transcription termination signal, that is, a terminator region, downstream of the nucleic acid having a nucleotide sequence or a portion thereof complementary to the nucleotide sequence that encodes the PCA-1 polypeptide. Furthermore, the expression vector can further comprise a selection marker gene (genes that confer resistance to drugs such as tetracycline, ampicillin, kanamycin, hygromycin, and phosphinothricin, genes that compensate for auxotrophic mutation, genes that encode fluorescent proteins, and the like).

An antibody that specifically recognizes the PCA-1 polypeptide is capable of suppressing a function of PCA-1 by binding specifically to the PCA-1 polypeptide. The antibody may be a polyclonal antibody or a monoclonal antibody, and can be prepared by a well-known immunological technique. The antibody may also be a bindable fragment of an antibody (for example, Fab, F(ab')₂), or a recombinant antibody (for example, single-chain antibody).

For example, the polyclonal antibody can be acquired by administering the PCA-1 polypeptide or a fragment thereof (as required, may be prepared as a complex crosslinked to a carrier protein such as bovine serum albumin or KLH (keyhole limpet hemocyanin)) as the antigen, along with a commercially available adjuvant (for example, Freund's complete or incomplete adjuvant), to an animal subcutaneously or intraperitoneally about 2 to 4 times at intervals of 2 to 3 weeks (the antibody titer of partially drawn serum has been determined by a known antigen-antibody reaction and its elevation has been confirmed in advance), collecting whole blood about 3 to about 10 days after final immunization, and purifying the antiserum. As the animal to receive the antigen, mammals such as rats, mice, rabbits, goat, guinea pigs, and hamsters can be mentioned.

The monoclonal antibody can be prepared by a cell fusion method (for example, Takeshi Watanabe, Saibou Yugouhou No Genri To Monokuronaru Koutai No Sakusei, edited by Akira Taniuchi and Toshitada Takahashi, "Monokuronaru Koutai To Gan - Kiso To Rinsho -", pages 2-14, Science Forum Shuppan, 1985). For example, the PCA-1 polypeptide or a fragment thereof, along with a commercially available adjuvant, is subcutaneously or intraperitoneally administered to a mouse 2 to 4 times, and about 3 days after final administration, the spleen or lymph nodes are collected, and leukocytes are collected. These leukocytes and myeloma cells (for example, NS-1, P3X63Ag8 and the like) are cell-fused to obtain a hybridoma that produces a monoclonal antibody that specifically recognizes the PCA-1 polypeptide. This cell fusion may be performed by the PEG method [J. Immunol. Methods, 81(2): 223-228 (1985)], or by the voltage pulse method [Hybridoma, 7(6): 627-633 (1988)].

A hybridoma that produces the desired monoclonal antibody can be selected by detecting an antibody that binds specifically to the antigen from the culture supernatant using a widely known EIA or RIA method and the like. Cultivation of the hybridoma that produces the monoclonal antibody can be performed in vitro, or in vivo such as in mouse or rat ascitic fluid, preferably in mouse ascitic fluid, and the antibody can be acquired from the culture supernatant of the hybridoma or the ascitic fluid of the animal.

In view of therapeutic efficacy and safety in humans, the antibody may be a chimeric antibody or a humanized or human type antibody. The chimeric antibody can be prepared with reference to, for example, "Jikken Igaku (extra issue), Vol.6, No.10, 1988", JP-B-H03-73280 and the like; the humanized antibody can be prepared with reference to, for example, JP-T-H04-506458, JP-A-S62-296890 and the like; the human antibody can be prepared with reference to, for example, "Nature Genetics, Vol.15, p.146-156, 1997", "Nature Genetics, Vol.7, p.13-21, 1994", JP-T-H04-504365, International Application Publication WO94/25585, "Nikkei Science, June issue, pp. 40 to 50, 1995", "Nature, Vol. 368, pp. 856-859, 1994", JP-T-H06-500233 and the like.

To suppress the function of PCA-1 more efficiently, as the antibody that specifically recognizes the PCA-1 polypeptide, an antibody can be selected that specifically recognizes a functional domain of the PCA-1 polypeptide (active site for repair of DNA and RNA alkylation damage and the like) to reduce the function accounted for by the domain.

A dominant negative mutant of the PCA-1 polypeptide refers to a mutant wherein a function (activity) of the PCA-1 polypeptide has been reduced by mutagenesis. The dominant negative mutant is capable of indirectly inhibiting a function (activity) of the PCA-1 polypeptide by competing with the PCA-1 polypeptide. The dominant negative mutant can be prepared by introducing a mutation to the nucleic acid that encodes the PCA-1 polypeptide. Examples of the mutation include amino acid mutations in a functional domain (active site for repair of DNA and RNA alkylation damage and the like) that result in a reduction in the function accounted for by the domain (for example, deletion, substitution, and addition of one or more amino acids). A dominant negative mutant can be prepared by a method known per se using PCR or a commonly known mutagenesis reagent.

A nucleic acid having the nucleotide sequence that encodes the above-described antibody that specifically recognizes the PCA-1 polypeptide or the dominant negative mutant of the PCA-1 polypeptide are also preferable as the compound that suppresses the expression or function of PCA-1. The nucleic acid can be provided in a manner functionally joined downstream of an appropriate promoter (for example, promoters capable of exhibiting promoter activity in cells (prostate cancer cells and the like) of the subject mammal) in an appropriate expression vector (for example, expression vectors capable of functioning in cells (prostate cancer cells and the like) of the recipient mammal).

As the expression vector and the promoter used, the same ones used in the above-described "expression vector capable of expressing a nucleic acid or a portion thereof having a nucleotide sequence complementary to the nucleotide sequence that encodes the PCA-1 polypeptide" can be used. The expression vector can comprise a transcription termination signal, that is, a terminator region, downstream of a nucleic acid having a nucleotide sequence that encodes an antibody that specifically recognizes the PCA-1 polypeptide or a dominant negative mutant of the PCA-1 polypeptide. Furthermore, the expression vector, like the above described "expression vector capable of expressing a nucleic acid having a nucleotide sequence or a portion thereof complementary to the nucleotide sequence, that encodes the PCA-1 polypeptide", can further comprise a selection marker gene.

The agent for use in the present invention can comprise, in addition to a compound that suppresses the expression or function of PCA-1, an optionally chosen carrier, for example, a pharmaceutically acceptable carrier.

As examples of the pharmaceutically acceptable carrier, excipients such as sucrose, starch, mannit, sorbit, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrants such as starch, carboxymethylcellulose, hydroxypropyl starch, sodium-glycol-starch, sodium hydrogen carbonate, calcium phosphate, and calcium citrate; lubricants such as magnesium stearate, Aerosil, talc, and sodium lauryl sulfate; flavoring agents such as citric acid, menthol, glycyrrhizin ammonium salt, glycine, and orange flour; preservatives such as sodium benzoate, sodium hydrogen sulfite, methylparaben, and propylparaben; stabilizers such as citric acid, sodium citrate, and acetic acid; suspending agents such as methylcellulose, polyvinylpyrrolidone, and aluminum stearate; dispersing agents such as surfactants; diluents such as water, physiological saline, and orange juice; base waxes such as cacao butter, polyethylene glycol, and kerosene; and the like can be mentioned, but these are not to be construed as limiting.

Preparations suitable for oral administration are liquids prepared by dissolving an effective amount of an active ingredient in a diluent such as water or physiological saline, capsules, saches or tablets containing an effective amount of an active ingredient in the form of solids or granules, suspensions prepared by suspending an effective amount of an active ingredient in an appropriate dispersant, emulsions prepared by dispersing and emulsifying a solution of an effective amount of an active ingredient in an appropriate dispersant, and the like.

Preparations suitable for parenteral administration (for example, subcutaneous injection, intramuscular injection, topical injection, intraperitoneal administration and the like) are aqueous and non-aqueous isotonic sterile injectable liquids, which may contain an antioxidant, a buffer solution, a bacteriostatic agent, an isotonizing agent and the like. Aqueous and non-aqueous sterile suspensions can also be mentioned, which may contain a suspending agent, a solubilizer, a thickening agent, a stabilizer, an antiseptic and the like. The preparation can be enclosed in containers such as ampoules and vials for unit dosage or a plurality of dosages. It is also possible to freeze-dry the active ingredient and a pharmaceutically acceptable carrier, and store them in a state that may be dissolved or suspended in an appropriate sterile vehicle just before use.

When the compound that suppresses the expression or function of PCA-1 is a nucleic acid, the agent for use in the present invention can further comprise a reagent for nucleic acid introduction in order to promote the introduction of the nucleic acid to the cells. When the nucleic acid is incorporated in a viral vector, particularly in retrovirus vector, retronectin, fibronectin, polybrene and the like can be used as the transfection reagent. When the nucleic acid is incorporated in a plasmid vector, cationic lipids such as lipofectin, lipfectamine, DOGS (transfectam; dioctadecylamidoglycylspermine), DOPE (1,2-dioleoyl-sn-glycero-3-phosphoethanolamine), DOTAP (1,2-dioleoyl-3-trimethylammoniumpropane), DDAB (dimethyldioctadecylammonium bromide), DHDEAB (N,N-di-n-hexadecyl-N,N-dihydroxyethylammonium bromide), HDEAB (N-n-hexadecyl-N,N-dihydroxyethylammonium bromide), polybrene, or poly(ethyleneimine) (PEI) can be used.

When the compound that suppresses the expression or function of PCA-1 is a polypeptide (antibody, dominant negative mutant and the like), the agent of the present invention can further comprise a reagent for polypeptide introduction in order to increase the efficiency of introduction of the polypeptide in the cells. As the reagent, Profect (produced by Nacalai Tesque, Inc.), ProVectin (produced by IMGENEX) and the like can be used.

The dosage of the agent used in the present invention varies depending on the activity and choice of the active ingredient, seriousness of the disease, animal species being the subject of administration, drug tolerance, body weight, and age of the subject of administration, and the like, and is generally about 0.0001 to about 5000 mg/kg, based on the amount of active ingredient per day for an adult.

In one embodiment, the agent used in the present invention can be for promoting apoptosis (apoptosis promoting agent). In this case, the apoptosis preferably is caspase 8/FADD dependent or induced by paclitaxel. As caspase 8/FADD-dependent apoptosis, FASL-induced apoptosis, TRAIL-induced apoptosis, TNF-α-induced apoptosis and the like can be mentioned. The reason why caspase 8/FADD-dependent apoptosis is preferred is that when the expression or function of PCA-1 is suppressed by an active ingredient of the agent of the present invention, the inhibitory function of PCA-1 on the ubiquitination of FLIP is suppressed, and the amount of FLIP polypeptide expressed in the cells decreases, whereby the suppression of caspase 8/FADD-dependent apoptosis by FLIP can be cancelled. The reason why paclitaxel-induced apoptosis is preferred is that paclitaxel-induced apoptosis is inhibited by the activation of MAP kinase that can be induced by paclitaxel per se (ERK and the like) (McDaid H.M. et al, Mol. Pharmacol., 60(2), 290-301, 2001), and that when the expression or function of PCA-1 is suppressed by an active ingredient of the agent of the present invention, the inhibitory function of PCA-1 on the ubiquitination of FLIP is suppressed, and the amount of FLIP polypeptide expressed in the cells decreases, whereby the interaction between FLIP and Raf-1 decreases, and the activation of Raf-1 is suppressed, and as a result, the activation of MAP kinase signaling can be suppressed

The apoptosis promoting agent used in the present invention is capable of promoting the apoptosis of cells (cells isolated from tissues (including cultured cells), normal cells, cancer cells, cell lines and the like), preferably prostate cancer cells, derived from a desired tissue (for example, prostate, thymus, liver, testis and the like) of one of the above-described mammals.

In one embodiment, the agent used in the present invention can be for inhibiting cell growth (cell growth inhibitor). When the expression or function of PCA-1 is suppressed by an active ingredient of the agent of the present invention, the inhibitory function of PCA-1 on the ubiquitination of FLIP is suppressed, and the amount of FLIP polypeptide expressed in the cells decreases, whereby the FLIP-Raf-1 interaction decreases, and as a result, MAP kinase signaling is suppressed; therefore, the agent of the present invention is capable of suitably inhibiting MAP kinase signaling-dependent cell growth. As examples of the MAP kinase signaling-dependent cell growth, cell growth dependent on a growth factor such as EGF and the like can be mentioned.

The cell growth inhibitor used in the present invention is capable of inhibiting the growth of cells (cells isolated from tissues (including cultured cells), normal cells, cancer cells, cell line and the like), preferably prostate cancer cells, derived from a desired tissue (for example, prostate, thymus, liver, testis and the like) of one of the above-described mammals.

In one embodiment, the agent used in the present invention can be for preventing/ treating cancer (prophylactic/therapeutic agent for cancer). In this case, the cancer can be a cancer derived from a desired tissue (for example, prostate, thymus, liver, testis and the like) of one of the above-described mammals, and is preferably prostate cancer. "prostate cancer" refers to a concept broadly involving the cancers that have developed in the prostate, and encompasses not only adenocarcinomas that have developed in the prostate, but also squamous cell carcinoma, transitional cell carcinoma, neuroendocrine carcinoma, undifferentiated cancers and the like. Preferably, the prostate cancer is an adenocarcinoma that has developed in the prostate. In prostate cancer, PCA-1 is highly expressed (Proceedings of the 123rd Annual Meeting of the Pharmaceutical Society of Japan No.4, p15, 2003), and the agent of the present invention can be particularly effective.

### (2. A screening method comprising selecting a compound that suppresses the expression or function of PCA-1)

As described above, a compound that suppresses the expression or function of PCA-1 is capable of inhibiting the anticancer-agent resistance of cancer cells to prevent/treat cancer by promoting apoptosis (caspase 8/FADD-dependent apoptosis, paclitaxel-induced apoptosis and the like) and inhibiting MAP kinase signaling-dependent cell growth (cell growth dependent on a growth factor such as EGF and the like). Therefore, by selecting a compound capable of suppressing the expression or function of PCA-1, a compound for promoting apoptosis, a compound for inhibiting cell growth, or a compound for preventing/ treating cancer can be acquired.

The test compound subjected to the screening method may be any commonly known compound or novel compound; for example, nucleic acids, carbohydrates, lipids, proteins, peptides, organic low molecular compounds, compound libraries prepared using combinatorial chemistry technology, random peptide libraries prepared by solid phase synthesis or the phage display method, or naturally occurring ingredients derived from microorganisms, animals, plants, marine organisms and the like, and the like can be mentioned.

For example, when a compound capable of suppressing the expression of PCA-1 is selected, a test compound and cells permitting a measurement of the expression of PCA-1 are brought into contact with each other, the amount of PCA-1 expressed in the cells brought into contact with the test compound is measured, and the amount expressed is compared with the amount of PCA-1 expressed in control cells not brought into contact with the test compound.

Cells permitting a measurement of the expression of PCA-1 refer to cells permitting a direct or indirect evaluation of the expression level of a product, for example, transcription product or translation product, of the PCA-1 gene. The cells permitting a direct evaluation of the expression level of a product of the PCA-1 gene can be cells capable of naturally expressing PCA-1; meanwhile, the cells permitting an indirect evaluation of the expression level of a product of the PCA-1 gene can be cells permitting reporter assay for the PCA-1 gene transcriptional regulatory region.

Cells capable of naturally expressing PCA-1 are not limited, as long as they potentially express PCA-1. These cells can easily be identified by those skilled in the art; useful cells include primary cultured cells, cell lines induced from the primary cultured cells, commercially available cell lines, cell lines available from cell banks, and the like. As examples of the cells capable of naturally expressing PCA-1, prostate cancer cells and the like of one of the aforementioned mammals can be mentioned.

The cells permitting reporter assay for the PCA-1 gene transcriptional regulatory region are cells comprising a PCA-1 gene transcriptional regulatory region and a reporter gene functionally joined to the region. The PCA-1 gene transcriptional regulatory region and the reporter gene can be inserted in an expression vector. The PCA-1 gene transcriptional regulatory region is not particularly limited, as long as it is a region capable of controlling the expression of the PCA-1 gene; for example, a region from the transcription initiation point to about 2 kbp upstream thereof, or a region consisting of the base sequence of the foregoing region wherein one or more bases have been deleted, substituted or added, and having the capability of controlling the transcription of the PCA-1 gene, and the like can be mentioned. The reporter gene may be any gene that encodes an enzyme that produces a detectable protein or a detactable substance; examples include the GFP (green fluorescent protein) gene, the GUS (β-glucuronidase) gene, the LUS (luciferase) gene, the CAT (chloramphenicol acetyltransferase) gene and the like.

The cells to be transfected with the PCA-1 gene transcriptional regulatory region and a reporter gene functionally joined to the region are not particularly limited, as long as they permit an evaluation of the PCA-1 gene transcriptional regulatory function, that is, as long as the amount of reporter gene expressed can be quantitatively analyzed. However, it is preferable that the above-described cells capable of naturally expressing the PCA-1 gene (for example, prostate cancer cells and the like) be used as the cells to be transfected because these cells express a physiological transcriptional regulatory factor for the PCA-1 gene, and are thought to be more suitable for the evaluation of the regulation of the expression of the PCA-1 gene.

Contact of a test compound with cells permitting a measurement of the expression of PCA-1 can be performed in an appropriate culture medium. The culture medium can be chosen as appropriate according to the choice of cells used and the like; for example, a minimal essential medium (MEM), Dulbecco's modified Eagle medium (DMEM), RPMI1640 medium, 199 medium and the like, containing about 5 to 20% fetal bovine serum, can be mentioned. Cultivation conditions are also determined as appropriate according to the choice of cells used and the like; for example, the pH of the medium is about 6 to about 8, cultivation temperature is normally about 30 to about 40°C, and cultivation time is about 12 to about 72 hours.

Next, the amount of PCA-1 expressed in the cells brought into contact with the test compound is measured. A measurement of the amount expressed can be performed by a method known per se, taking into consideration the kind of cell used and the like. For example, when cells capable of naturally expressing PCA-1 are used as the cells permitting a measurement of the expression of PCA-1, the amount expressed can be measured with a product, for example, transcription product (mRNA) or translation product (polypeptide), of the PCA-1 gene as the subject of measurement, by a method known per se. For example, the amount of transcription product expressed can be measured by preparing total RNA from the cells, and performing RT-PCR, Northern blotting and the like. The amount of translation product expressed can be measured by preparing an extract from the cells, and performing an immunological technique. As the immunological technique, radioisotope immunoassay (RIA), ELISA (Methods in Enzymol. 70: 419-439 (1980)), the fluorescent antibody method, Western blotting and the like can be used. Meanwhile, when cells permitting reporter assay of the PCA-1 gene transcriptional regulatory region are used as the cells permitting a measurement of the expression of PCA-1, the amount expressed can be measured on the basis of the signal intensity of the reporter.

Next, the amount of PCA-1 expressed in the cells brought into contact with the test compound is compared with the amount of PCA-1 expressed in control cells not brought into contact with the test compound. This comparison of the amount expressed is performed preferably on the basis of the presence or absence of a significant difference. Although the amount of PCA-1 expressed in the control cells not brought into contact with the test compound may be measured before, or simultaneously with, the measurement of the amount of PCA-1 expressed in the cells brought into contact with the test compound, it is preferable, from the viewpoint of experimental accuracy and reproducibility, that the amount expressed be simultaneously measured.

As a result of the comparison, a compound judged to be capable of suppressing the expression of PCA-1 can be obtained as a compound for promoting apoptosis, a compound for inhibiting cell growth, or a compound for preventing or treating cancer.

When a compound capable of suppressing a function of PCA-1 is chosen, the function (activity) of PCA-1 is measured in the presence of a test compound, and the function (activity) is compared with the function (activity) of PCA-1 in the absence of the test compound.

For example, when a function to inhibit the ubiquitination of FLIP is measured as the function of PCA-1, cells permitting a measurement of the FLIP ubiquitination inhibitory activity of PCA-1 and a test compound are brought into contact with each other, the FLIP ubiquitination inhibitory activity in the cells brought into contact with the test compound is measured, and this is compared with the FLIP ubiquitination inhibitory activity in control cells not brought into contact with the test compound. In this case, for the purpose of suppressing the degradation of ubiquitinated FLIP by proteasome, the cells and the test compound may be brought into contact with each other in the presence of a proteasome inhibitor (for example, MG132 and the like).

The cells used are not particularly limited, as long as they permit a measurement of the FLIP ubiquitination inhibitory activity of PCA-1; for example, cells that express PCA-1, FLIP, and an enzyme involved in FLIP ubiquitination and the like in a functional manner and the like can be mentioned. The PCA-1, FLIP, and enzyme involved in FLIP ubiquitination and the like may be naturally expressed ones, or ones forcibly expressed by transfection. As examples of cells permitting a measurement of the FLIP ubiquitination inhibitory activity of PCA-1, cells obtained by introducing the PCA-1 gene to cells wherein the occurrence of FLIP ubiquitination has been confirmed, and having the FLIP ubiquitination inhibited by the introduced PCA-1, and the like can be mentioned. "Cells wherein the occurrence of FLIP ubiquitination has been confirmed" can easily be identified by those skilled in the art; for example, useful cells include primary culture cells of the above-described mammals, cell lines induced from the primary culture cells, commercially available cell lines, cell lines available from cell banks and the like. The "cells wherein the occurrence of FLIP ubiquitination has been confirmed" are preferably prostate cancer cells.

Contact of a test compound with cells permitting a measurement of the FLIP ubiquitination inhibitory activity of PCA-1, like contact of a test compound with cells permitting a measurement of PCA-1 expression, can be performed in an appropriate culture medium (for example, a minimal essential medium (MEM), Dulbecco's modified Eagle medium (DMEM), RPMI1640 medium and 199 medium, containing about 5 to 20% fetal bovine serum). Cultivation conditions are determined as appropriate according to the choice of cells used, the choice of interaction measured and the like; for example, the pH of the medium is about 6 to about 8, cultivation temperature is normally about 30 to about 40°C, and cultivation time is about 1 hour to about 72 hours.

Next, the FLIP ubiquitination inhibitory activity of PCA-1 in the cells brought into contact with the test compound is measured. A measurement of FLIP ubiquitination can be performed by a method known per se; for example, an evaluation can be achieved by immunoprecipitating FLIP from an extract from the cells, and quantifying the amount of ubiquitin bound to the FLIP by Western blotting. In this case, it is judged that the lower the degree of FLIP ubiquitination is, the higher the "FLIP ubiquitination inhibitory activity of PCA-1" is; when the "FLIP ubiquitination inhibitory activity of PCA-1" is suppressed, the degree of FLIP ubiquitination increases.

Next, the FLIP ubiquitination inhibitory activity of PCA-1 in the cells brought into contact with the test compound is compared with the activity in control cells not brought into contact with the test compound. This comparison of the amount expressed is performed preferably on the basis of the presence or absence of a significant difference. Although the FLIP ubiquitination inhibitory activity of PCA-1 in the control cells not brought into contact with the test compound may be measured before, or simultaneously with, the measurement of the FLIP ubiquitination inhibitory activity of PCA-1 in the cells brought into contact with the test compound, it is preferable, from the viewpoint of experimental accuracy and reproducibility, that the activity be simultaneously measured.

When a function to increase the FLIP-Raf-1 interaction is measured as the function of PCA-1, cells permitting a measurement of the FLIP-Raf-1 interaction-increasing activity of PCA-1 and a test compound are brought into contact with each other, the FLIP-Raf-1 interaction-increasing activity in the cells brought into contact with the test compound is measured, and this is compared with the FLIP-Raf-1 interaction-increasing activity in control cells not brought into contact with the test compound.

The cells used are not particularly limited, as long as they permit a measurement of the FLIP-Raf-1 interaction-increasing activity of PCA-1; for example, cells that express PCA-1, FLIP, and Raf-1 in a functional manner and the like can be mentioned. The PCA-1, FLIP, Raf-1 and the like may be naturally expressed ones, or ones forcibly expressed by transfection. As examples of cells permitting a measurement of the FLIP-Raf-1 interaction-increasing activity of PCA-1, cells obtained by introducing the PCA-1 gene to cells wherein the occurrence of FLIP-Raf-1 interaction has been confirmed, and having the FLIP-Raf-1 interaction increased by the introduced PCA-1 and the like can be mentioned. "Cells wherein the occurrence of FLIP-Raf-1 interaction has been confirmed" can easily be identified by those skilled in the art; for example, useful cells include primary culture cells of the above-described mammals, cell lines induced from the primary culture cells, commercially available cell lines, cell lines available from cell banks and the like. The "cells wherein the occurrence of FLIP-Raf-1 interaction has been confirmed" are preferably prostate cancer cells.

Contact of a test compound with cells permitting a measurement of the FLIP-Raf-1 interaction-increasing activity of PCA-1, like that described above, can be performed in an appropriate culture medium (for example, a minimal essential medium (MEM), Dulbecco's modified Eagle medium (DMEM), RPMI1640 medium and 199 medium, containing about 5 to 20% fetal bovine serum). Cultivation conditions are determined as appropriate according to the choice of cells used, the choice of interaction determined and the like; for example, the pH of the medium is about 6 to about 8, cultivation temperature is normally about 30 to about 40°C, and cultivation time is about 1 minute to about 72 hours.

Next, the FLIP-Raf-1 interaction-increasing activity of PCA-1 in the cells brought into contact with the test compound is measured. A measurement of FLIP-Raf-1 interaction can be performed by a method known per se; for example, an evaluation can be achieved by immunoprecipitating FLIP from an extract from the cells, and quantifying the amount of Raf-1 co-precipitated with the FLIP by Western brotting. In this case, it is judged that the higher the degree of FLIP-Raf-1 interaction is, the higher "the FLIP-Raf-1 interaction-increasing activity of PCA-1" is; when "the FLIP-Raf-1 interaction-increasing activity of PCA-1" is suppressed, the degree of FLIP-Raf-1 interaction decreases.

Next, the FLIP-Raf-1 interaction-increasing activity of PCA-1 in the cells brought into contact with the test compound is compared with the activity in the control cells not brought into contact with the test compound. This comparison of the activity can be performed preferably on the basis of the presence or absence of a significant difference. Although the FLIP-Raf-1 interaction-increasing activity of PCA-1 in the control cells not brought into contact with the test compound may be measured before, or simultaneously with, the measurement of the FLIP-Raf-1 interaction-increasing activity of PCA-1 in the cells brought into contact with the test compound, it is preferable, from the viewpoint of experimental accuracy and reproducibility, that the activity be simultaneously measured.

When the function to enhance MAP kinase signaling is measured as the function of PCA-1, cells permitting a measurement of the MAP kinase signaling-enhancing activity of PCA-1 and a test compound are brought into contact with each other, the MAP kinase signaling-enhancing activity in the cells brought into contact with the test compound is measured, and this is compared with the MAP kinase signaling-enhancing activity in control cells not brought into contact with the test compound.

The cells used are not particularly limited, as long as they permit a measurement of the MAP kinase signaling-enhancing activity of PCA-1; for example, cells that express PCA-1 and a molecule that constitutes MAP kinase signaling (Raf-1, MEK1, ERK and the like) in a functional manner and the like can be mentioned. The PCA-1 and the molecule that constitutes MAP kinase signaling may be naturally expressed ones, or ones forcibly expressed by transfection. As examples of the cells permitting a measurement of the MAP kinase signaling-enhancing activity of PCA-1, cells obtained by introducing the PCA-1 gene to cells wherein the occurrence of MAP kinase signaling has been confirmed, and having the MAP kinase signaling enhanced by the introduced PCA-1, and the like can be mentioned. "Cells wherein the occurrence of MAP kinase signaling has been confirmed" can easily be identified by those skilled in the art; for example, useful cells include primary cultured cells of the above-described mammals, cell lines induced from the primary cultured cells, commercially available cell lines, cell lines available from cell banks and the like. The "cells wherein the occurrence of MAP kinase signaling has been confirmed" are preferably prostate cancer cells. Because MAP kinase signaling generally depends on cell activation, the cells may be activated by being treated as appropriate. For example, when prostate cancer cells and the like are used, the cells can be stimulated with a growth factor capable of activating MAP kinase signaling, such as EGF.

Contact of a test compound with cells permitting a measurement of the MAP kinase signaling-enhancing activity of PCA-1, like that described above, can be performed in an appropriate culture medium (for example, a minimal essential medium (MEM), Dulbecco's modified Eagle medium (DMEM), RPMI1640 medium, 199 medium, containing about 5 to 20% fetal bovine serum). Cultivation conditions are determined as appropriate according to the choice of cells used, the choice of interaction determined and the like; for example, the pH of the medium is about 6 to about 8, cultivation temperature is normally about 30 to about 40°C, and cultivation time is about 1 minute to about 72 hours.

Next, the MAP kinase signaling-enhancing activity of PCA-1 in the cells brought into contact with the test compound is measured. A measurement of MAP kinase signaling can be performed by a method known per se, normally with the phosphorylation of a molecule that constitutes MAP kinase signaling (Raf-1, MEK1, ERK and the like) as an index. For example, MAP kinase signaling can be measured by using cells cultured in the presence of γ³²P-ATP, with an antibody against the molecule that constitutes MAP kinase signaling (Raf-1, MEK1, ERK and the like) and the like, immunoprecipitating the molecule that constitutes MAP kinase signaling from an extract of the cells, and quantifying the amount of ³²P incorporated in the molecule by phosphorylation. Using an antibody specific for the molecule that constitutes MAP kinase signaling, the degree of phosphorylation may be measured by Western blotting and the like. In this case, it is judged that the higher the degree of phosphorylation of the molecule that constitutes MAP kinase signaling is, the higher "the MAP kinase signaling-enhancing activity of PCA-1" is; when "the MAP kinase signaling-enhancing activity of PCA-1" is suppressed, the degree of phosphorylation of the molecule that constitutes MAP kinase signaling decreases.

Next, the MAP kinase signaling-enhancing activity of PCA-1 in the cells brought into contact with the test compound is compared with the activity in the control cells not brought into contact with the test compound. This comparison of the activity is performed preferably on the basis of the presence or absence of a significant difference. Although the MAP kinase signaling-enhancing activity of PCA-1 in the control cells not brought into contact with the test compound may be measured before, or simultaneously with, the measurement of the MAP kinase signaling-enhancing activity of PCA-1 in the cells brought into contact with the test compound, it is preferable, from the viewpoint of experimental accuracy and reproducibility, that the activity be simultaneously measured.

Next, the MAP kinase signaling-enhancing activity of PCA-1 in the cells brought into contact with the test compound is compared with the activity in the control cells not brought into contact with the test compound. This comparison of the activity is performed preferably on the basis of the presence or absence of a significant difference. Although the MAP kinase signaling-enhancing activity of PCA-1 in the control cells not brought into contact with the test compound may be measured before, or simultaneously with, the measurement of the MAP kinase signaling-enhancing activity of PCA-1 in the cells brought into contact with the test compound, it is preferable, from the viewpoint of experimental accuracy and reproducibility, that the activity be simultaneously measured.

When a function to increase cyclin D1 expression is measured as the function of PCA-1, cells permitting a measurement of the cyclin D1 expression-increasing activity of PCA-1 and a test compound are brought into contact with each other, the cyclin D1 expression-increasing activity in the cells brought into contact with the test compound is measured, and this is compared with the cyclin D1 expression-increasing activity in control cells not brought into contact with the test compound.

The cells used are not particularly limited, as long as the cyclin D1 expression-increasing activity of PCA-1 can be measured; for example, cells that express PCA-1 and cyclin D1 in a functional manner and the like can be mentioned. The PCA-1 may be naturally expressed one, or one forcibly expressed by transfection. The cyclin D1 is preferably a naturally expressible one. As examples of the cells permitting a measurement of the cyclin D1 expression-increasing activity of PCA-1, cells obtained by introducing the PCA-1 gene to cells wherein the potential for cyclin D1 expression has been confirmed, and having cyclin D1 expression enhanced by the introduced PCA-1, and the like can be mentioned. "Cells wherein the potential for cyclin D1 expression has been confirmed" can easily be identified by those skilled in the art; for example, useful cells include primary culture cells of the above-described mammals, cell lines induced from the primary culture cells, commercially available cell lines, cell lines available from cell banks and the like. The "cells wherein the potential for cyclin D1 expression has been confirmed" are preferably prostate cancer cells. Because cyclin D1 expression normally depends on cell activation (particularly activation of MAP kinase signaling), the cells may be activated by being treated as appropriate. For example, when prostate cancer cells and the like are used, the cells can be stimulated with a growth factor capable of activating MAP kinase signaling, such as EGF.

Contact of a test compound with cells permitting a measurement of the cyclin D1 expression-increasing activity of PCA-1, like that described above, can be performed in an appropriate culture medium (for example, a minimal essential medium (MEM), Dulbecco's modified Eagle medium (DMEM), RPMI1640 medium and 199 medium, containing about 5 to 20% fetal bovine serum). Cultivation conditions are determined as appropriate according to the choice of cells used, the choice of interaction determined and the like; for example, the pH of the medium is about 6 to about 8, cultivation temperature is normally about 30 to about 40°C, and cultivation time is about 1 minute to about 72 hours.

Next, the cyclin D1 expression-increasing activity of PCA-1 in the cells brought into contact with the test compound is measured. The amount of cyclin D1 expressed can be measured with a product, for example, transcription product (mRNA) or translation product (polypeptide), of the cyclin D1 gene as the subject of measurement, by a method known per se. For example, the amount of transcription product expressed can be measured by preparing total RNA from the cells, and performing RT-PCR, Northern blotting and the like. The amount of translation product expressed can be measured by preparing an extract from the cells, and performing an immunological technique. As the immunological technique, radioisotope immunoassay (RIA), ELISA (Methods in Enzymol. 70: 419-439 (1980)), the fluorescent antibody method, Western blotting and the like can be used. In this case, it is judged that the larger the amount of cyclin D1 expressed is, the higher "the cyclin D1 expression-increasing activity of PCA-1" is; if "the cyclin D1 expression-increasing activity of PCA-1" is suppressed, the degree of the amount of cyclin D1 expressed decreases.

Next, the cyclin D1 expression-increasing activity of PCA-1 in the cells brought into contact with the test compound is compared with the activity in the control cells not brought into contact with the test compound. This comparison of the activity is performed preferably on the basis of the presence or absence of a significant difference. Although the cyclin D1 expression-increasing activity of PCA-1 in the control cells not brought into contact with the test compound may be measured before, or simultaneously with, the measurement of the cyclin D1 expression-increasing activity of PCA-1 in the cells brought into contact with the test compound, it is preferable, from the viewpoint of experimental accuracy and reproducibility, that the activity be simultaneously measured.

As a result of the comparison, a compound judged to be capable of suppressing the function of PCA-1 can be selected as a compound for promoting apoptosis, a compound for inhibiting cell growth, or a compound for preventing or treating cancer.

In the screening method of the present invention, particularly when a compound for promoting apoptosis is screened for, the apoptosis can preferably be caspase 8/FADD dependent or induced by paclitaxel. As the caspase 8/FADD-dependent apoptosis, FASL-induced apoptosis, TRAIL-induced apoptosis, TNF-α-induced apoptosis and the like can be mentioned. The reason why caspase 8/FADD-dependent apoptosis is preferred is that when the expression or function of PCA-1 is suppressed by an active ingredient of the agent of the present invention, the inhibitory function of PCA-1 on the ubiquitination of FLIP is suppressed, and the amount of FLIP polypeptide expressed in the cells decreases, whereby the suppression of caspase 8/FADD-dependent apoptosis by FLIP can be cancelled. The reason why caspase paclitaxel-induced apoptosis is preferred is that paclitaxel-induced apoptosis is inhibited by the activation of MAP kinase (ERK and the like), which can be induced by paclitaxel per se (McDaid H.M. et al, Mol. Pharmacol., 60(2), 290-301, 2001), and that when the expression or function of PCA-1 is suppressed by an active ingredient of the agent of the present invention, the inhibitory function of PCA-1 on the ubiquitination of FLIP is suppressed, and the amount of FLIP polypeptide expressed in the cells decreases, whereby the interaction between FLIP and Raf-1 decreases, the activation of Raf-1 is suppressed, and as a result, the activation of MAP kinase signaling can be suppressed.

Taking into consideration the above-described mechanism of the involvement of PCA-1 in apoptosis, when a compound for promoting apoptosis is screened for by selecting a compound capable of suppressing the function of PCA-1 in the method of the present invention, a function of PCA-1 can preferably be selected from the group consisting of (i) to (iii) below:
(i) a function to inhibit the ubiquitination of FLIP;
(ii) a function to increase the FLIP-Raf-1 interaction;
(iii) a function to enhance MAP kinase signaling.

When the expression or function of PCA-1 is suppressed, the inhibitory function of PCA-1 on the ubiquitination of FLIP is suppressed, the ubiquitination of FLIP is enhanced, and the amount of FLIP polypeptide expressed in the cells decreases, whereby the FLIP-Raf-1 interaction decreases, and as a result, MAP kinase signaling weakens, cyclin D1 expression is suppressed, and finally cell growth is inhibited. Therefore, taking into consideration this mechanism of action of PCA-1, particularly when a compound for inhibiting cell growth is screened for in the method of the present invention, the cell growth can preferably depend on MAP kinase signaling. As examples of MAP kinase signaling-dependent cell growth, cell growth depending on a cell growth factor such as EGF and the like can be mentioned.

Taking into consideration the above-described mechanism of the involvement of PCA-1 in cell growth, when a compound for inhibiting cell growth is screened for by selecting a compound capable of suppressing the function of PCA-1 in the method of the present invention, a function of PCA-1 can preferably be selected from the group consisting of (i) to (iv) below:
(i) a function to inhibit the ubiquitination of FLIP;
(ii) a function to increase the FLIP-Raf-1 interaction;
(iii) a function to enhance MAP kinase signaling;
(iv) a function to increase cyclin D expression.

In the screening method of the present invention, particularly when a compound for preventing/ treating cancer is screened for, the cancer can be a cancer derived from a desired tissue (for example, prostate, thymus, liver, testis and the like) of one of the above-described mammals, and is preferably prostate cancer. In prostate cancer, PCA-1 is highly expressed (Proceedings of the 123rd Annual Meeting of the Pharmaceutical Society of Japan No.4, p15, 2003), and a compound obtained by the screening method of the present invention can be particularly effective.

Taking into consideration the above-described mechanism of the involvement of PCA-1 in apoptosis and cell growth, when a compound for preventing/ treating cancer is screened for by selecting a compound capable of suppressing the function of PCA-1 in the method of the present invention, a function of PCA-1 can preferably be selected from the group consisting of (i) to (iv) below:
(i) a function to inhibit the ubiquitination of FLIP;
(ii) a function to increase the FLIP-Raf-1 interaction;
(iii) a function to enhance MAP kinase signaling;
(iv) a function to increase cyclin D expression.

A compound that can be obtained by the screening method of the present invention can be rendered a candidate compound for pharmaceutical development, and, like the above-described agent used in the present invention, can be prepared as an apoptosis promoting agent, a cell growth inhibitor, or a prophylactic/therapeutic agent for cancer (prostate cancer and the like).

### (3. A screening method for a compound for promoting the ubiquitination of FLIP)

As shown in an Example below, when PCA-1 is highly expressed, the ubiquitination of FLIP is inhibited, the expression of FLIP polypeptide increases, and as a result, apoptosis is inhibited, cell growth is enhanced, and the cells become resistant to anticancer agents. Therefore, a compound capable of promoting the ubiquitination of FLIP in cells wherein PCA-1 is expressed, if obtained, would be useful as a compound for promoting apoptosis, a compound for inhibiting cell growth, and a compound for preventing/ treating cancer. In particular, in prostate cancer, because PCA-1 is highly expressed (Proceedings of the 123rd Annual Meeting of the Pharmaceutical Society of Japan No.4, p15, 2003), a compound capable of promoting the ubiquitination of FLIP in cells wherein PCA-1 is expressed can be particularly useful as a compound for preventing/ treating prostate cancer. Accordingly, the present invention provides an in vitro screening method for a compound for promoting the ubiquitination of FLIP, comprising the following steps:
(1) a step for treating PCA-1-expressing cells with a test compound in the presence of a proteasome inhibitor;
(2) a step for evaluating the ubiquitination of FLIP in the aforementioned cells.

PCA-1-expressing cells can easily be identified by those skilled in the art; for example, useful cells include primary cultured cells of the above-described mammals, cell lines induced from the primary cultured cells, commercially available cell lines, cell lines available from cell banks and the like, and prostate cancer cells are preferably used. The amount of PCA-1 expressed in the cells is not particularly limited, as long as it is sufficient to inhibit the ubiquitination of FLIP; it is preferable that the amount expressed be sufficient to inhibit the ubiquitination of FLIP to an about 1/2, preferably about 1/10, level compared with cells that do not express PCA-1 (for example, cells wherein the functional expression of PCA-1 is inhibited by siRNA against PCA-1) in order to more securely inhibit the ubiquitination of FLIP. In the cells, the PCA-1 may be a naturally expressed one, or one forcibly expressed by transfection; however, it is preferable that the PCA-1 be forcibly expressed by transfection in order to achieve more potent and more secure PCA-1 expression. PCA-1 expression by transfection is achieved by introducing an expression vector capable of expressing PCA-1 to the above-described cells. The expression vector can be provided in a manner wherein the nucleic acid that encodes PCA-1 is functionally joined downstream of a promoter capable of functioning in the cells to be transfected.

In the step (1), PCA-1-expressing cells are treated with a test compound in the presence of a proteasome inhibitor. The test compound is the same as that used in the foregoing section (2. A screening method comprising selecting a compound capable of suppressing the expression or function of PCA-1). The reason why a proteasome inhibitor is used is that by suppressing the degradation of ubiquitinated FLIP by proteasome, the promoting activity of the test compound on the ubiquitination of FLIP is accurately evaluated. As the proteasome inhibitor, MG132 and the like can be mentioned.

Treatment of PCA-1-expressing cells with a test compound can be performed in an appropriate culture medium. The culture medium can be chosen as appropriate according to the choice of cells used and the like; for example, a minimal essential medium (MEM), Dulbecco's modified Eagle medium (DMEM), RPMI1640 medium, 199 medium and the like, containing about 5 to 20% fetal bovine serum can be mentioned. Cultivation conditions are also determined as appropriate according to the choice of cells used and the like; for example, the pH of the medium is about 6 to about 8, cultivation temperature is normally about 30 to about 40°C, and cultivation time is about 12 to about 72 hours. The proteasome inhibitor concentration in the medium is not particularly limited, as long as the degradation of ubiquitinated FLIP by proteasome is inhibited; for example, when MG132 is used, the concentration is normally about 10 to about 30 µM.

Next, the FLIP ubiquitination in the cells brought into contact with the test compound is evaluated. A measurement of the FLIP ubiquitination can be performed by a method known per se; for example, an evaluation can be achieved by immunoprecipitating FLIP from an extract of the cells, and quantifying the amount of ubiquitin bound to the FLIP by Western blotting.

Next, the FLIP ubiquitination in the cells brought into contact with the test compound is compared with the FLIP ubiquitination in the control cells not brought into contact with the test compound. This comparison of the FLIP ubiquitination is performed preferably on the basis of the presence or absence of a significant difference. Although the FLIP ubiquitination in the control cells not brought into contact with the test compound may be measured before, or simultaneously with, the measurement of the FLIP ubiquitination in the cells brought into contact with the test compound, it is preferable, from the viewpoint of experimental accuracy and reproducibility, that the amount expressed be simultaneously measured.

As a result of the comparison, a compound judged to have increased the degree of FLIP ubiquitination compared with the control cells is selected as a compound for promoting FLIP ubiquitination.

A compound that can be obtained by the screening method of the present invention can be rendered a candidate compound for pharmaceutical development, and, like the above-described agent for use in the present invention, can be prepared as an FLIP ubiquitination promoting agent, an apoptosis suppressing agent, a cell growth inhibitor, or a prophylactic/therapeutic agent for cancer (prostate cancer and the like).

### (4. Screening method for a compound for promoting apoptosis)

As shown in an Example below and the like, when PCA-1 is highly expressed, paclitaxel-induced apoptosis is inhibited, and resistance to paclitaxel is acquired. When PCA-1 is highly expressed, caspase 8/FADD-dependent apoptosis induced by TRAIL and the like is inhibited, and resistance to caspase 8/FADD-dependent apoptosis inducers (FASL, TRAIL, TNF-R, anti-FAS antibody and the like) is acquired. Therefore, a compound capable of promoting apoptosis induced by paclitaxel or a caspase 8/FADD-dependent apoptosis inducer in cells wherein PCA-1 is expressed, if obtained, would be useful as a compound for preventing/ treating cancer that has acquired resistance to paclitaxel or the caspase 8/FADD-dependent apoptosis inducer. In particular, in prostate cancer, because PCA-1 is highly expressed (Proceedings of the 123rd Annual Meeting of the Pharmaceutical Society of Japan No.4, p15, 2003), a compound capable of promoting apoptosis induced by paclitaxel or a caspase 8/FADD-dependent apoptosis inducer is useful as a compound for prostate cancer prophylaxis/treatment. Accordingly, the present invention provides an in vitro screening method for a compound for preventing or treating apoptosis, comprising the following steps:
(1) a step for treating PCA-1-expressing cells with paclitaxel in the presence of a test compound;
(2) a step for evaluating the degree of the apoptosis of the aforementioned cells;

Also described herein is a screening method for a compound for promoting caspase 8/FADD-dependent apoptosis, comprising the following steps:
(1) a step for treating PCA-1-expressing cells with paclitaxel or a caspase 8/FADD-dependent apoptosis inducer in the presence of a test compound;
(2) a step for evaluating the degree of the apoptosis of the aforementioned cells.

PCA-1-expressing cells can easily be identified by those skilled in the art; for example, useful cells include primary cultured cells of the above-described mammals, cell lines induced from the primary cultured cells, commercially available cell lines, cell lines available from cell banks and the like, and prostate cancer cells are preferably used. The amount of PCA-1 expressed in the cells is not limited, as long as it is sufficient to inhibit paclitaxel-induced apoptosis or caspase 8/FADD-dependent apoptosis; it is preferable that the amount expressed be sufficient to inhibit paclitaxel-induced apoptosis or caspase 8/FADD-dependent apoptosis to an about 1/2, preferably about 1/10, level compared with cells that do not express PCA-1 (for example, cells wherein the functional expression of PCA-1 is inhibited by siRNA against PCA-1) in order to construct a more sensitive screening method by securely inhibiting the apoptosis. In the cells, the PCA-1 may be naturally expressed one, or one forcibly expressed by transfection; however, it is preferable that the PCA-1 be forcibly expressed by transfection in order to achieve more potent and more secure PCA-1 expression. PCA-1 expression by transfection is achieved by introducing an expression vector capable of expressing PCA-1 to the above-described cells. The expression vector can be provided in a manner wherein the nucleic acid that encodes PCA-1 is functionally joined downstream of a promoter capable of functioning in the cells to be transfected.

In the step (1), PCA-1-expressing cells are treated with paclitaxel or a caspase 8/FADD-dependent apoptosis inducer in the presence of a test compound. The test compound is the same as used in the foregoing section (2. A screening method comprising selecting a compound capable of suppressing the expression or function of PCA-1).

Treatment of PCA-1-expressing cells with a test compound can be performed in an appropriate culture medium. The culture medium can be chosen as appropriate according to the choice of cells used and the like; for example, a minimal essential medium (MEM), Dulbecco's modified Eagle medium (DMEM), RPMI1640 medium, 199 medium and the like, containing about 5 to 20% fetal bovine serum can be mentioned. Cultivation conditions are also determined as appropriate according to the choice of cells used and the like; for example, the pH of the medium is about 6 to about 8, cultivation temperature is normally about 30 to about 40°C, and cultivation time is about 12 to about 72 hours. The paclitaxel concentration in the medium is not particularly limited, as long as it falls in a range of concentration allowing the induction of apoptosis in cells that are not expressing PCA-1, and the concentration is, for example, about 0.1 to about 20 µM. The concentration of caspase 8/FADD-dependent apoptosis inducer in the medium is not particularly limited, as long as it falls in a range of concentration allowing the induction of apoptosis in cells that are not expressing PCA-1, and the concentration is chosen as appropriate according to the choice of inducer and the choice of cells used for the screening; for example, when TRAIL is used as the caspase 8/FADD-dependent apoptosis inducer, the TRAIL concentration in the medium is about 1 to about 1000 ng/ml.

Next, the degree of apoptosis in the cells brought into contact with the test compound is evaluated. A measurement of apoptosis can be performed by a method known per se; for example, agarose gel electrophoresis for detecting a fragment cleaved in DNA nucleosome units as a "DNA ladder", pulse field electrophoresis for detecting apoptosis that produces 50 to 300 kbp high molecular weight DNA fragments, the in situ end labeling method (TUNEL method) for detecting a DNA cleavage end to detect apoptosis in tissue, and a method comprising staining cells with a fluorescent dye, and thereafter performing the detection of cell size change and cells with decreased DNA contents or detection of live or dead cells, and the like, by flowcytometry, and the like can be used.

Next, the degree of apoptosis in the cells brought into contact with the test compound is compared with the degree of apoptosis in control cells not brought into contact with the test compound. This comparison of the degree of apoptosis is performed preferably on the basis of the presence or absence of a significant difference. Although the apoptosis in the control cells not brought into contact with the test compound may be measured before, or simultaneously with, the measurement of the apoptosis in the cells brought into contact with the test compound, it is preferable, from the viewpoint of experimental accuracy and reproducibility, that the value be simultaneously measured.

As a result of the comparison, a compound judged to have increased the degree of paclitaxel-induced or caspase 8/FADD-dependent apoptosis compared with the control cells is selected as a compound for promoting paclitaxel-induced apoptosis or a compound for promoting caspase 8/FADD-dependent apoptosis.

A compound that can be obtained by the screening method of the present invention can be rendered a candidate compound for pharmaceutical development, and, like the above-described agent for use in the present invention, can be prepared as a paclitaxel-induced apoptosis promoting agent, a caspase 8/FADD-dependent apoptosis promoting agent, or a prophylactic/therapeutic agent for cancer (prostate cancer and the like).

The present invention is hereinafter described in more detail by means of the following Examples, which, however, do not limit the scope of the present invention by any means.

### Examples

### Example 1

### (Materials and methods)

### 1) Preparation of cell line, gene, and transfected cell line

The human prostate cancer cell line DU145 (purchased from ATCC) was cultured in RPMI medium (in the presence of 10% serum) and used. The human PCA-1 gene was cleaved out from a PCA-1 vector, and this was inserted into pIRES/Neo, which is a vector that encodes the neomycin resistance gene (hereinafter pIRES/Neo/PCA-1). DU145 was transfected (lipofection method) with this gene; 48 hours later, neomycin was added, the cells were cultured for about 2 months, and at least two clones exhibiting resistance to neomycin (DU145/PCA-1) were isolated and subjected to experiments that followed. For control, a clone (DU145/Neo) incorporating an empty vector, and cultured under the same conditions, was used.

Regarding FLIP (FLICE-like inhibitory protein), a cDNA was prepared by RT-PCR, inserted into pME18S-FLAG2, and DU145 was transfected (lipofection method) with the vector along with the pTK-Hyg vector (Clonetech Laboratories Japan, Ltd., Tokyo, Japan), which is a vector that encodes the hygromycin resistance gene. The cells were cultured in the presence of hygromycin for about 2 months, and at least two clones exhibiting resistance to hygromycin (DU145/FLIP) were isolated and subjected to experiments that followed. For control, a clone (DU145/HygB) incorporating an empty vector, and cultured under the same conditions, was used.

### 2) Knockdown of PCA-1

siRNA against PGA-1 (CCGAGAGUGAACCUGACC: SEQ ID NO:3) was inserted into the pRNA-H1.1/Hygro vector, and DU145 was transiently transfected with the vector (lipofection method). Expression suppressive and knockdown effects were confirmed by Western blotting.

### 3) FLIP ubiquitination assay

After stimulation with MG132, the cells were harvested and immunoprecipitated with an anti-FLIP antibody; thereafter, Western blot was performed using the anti-FLIP antibody and an anti-ubiquitin antibody, and the ubiquitination of the FLIP molecule was examined.

### 4) Search for cell growth

Experiments were performed using the MTS [(3-(4,5-dimethylthiazol-2-yl)-5-(3-carboxymethoxyphenyl)-2-(4-sulphonyl)-2H-tetrazolium, inner salt] reagent (Promega, Tokyo, Japan) in accordance with the attached protocol.

### (Results)

### 1) Function of PCA-1 on cell growth via epidermal growth factor

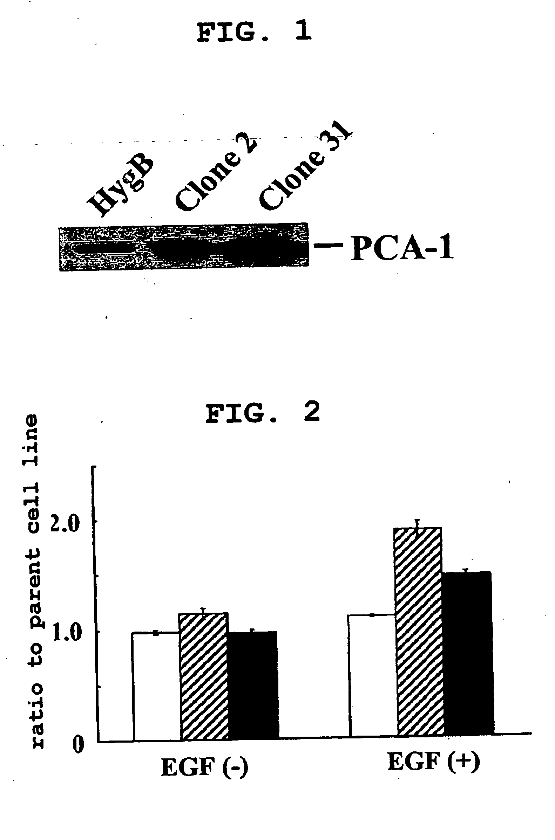

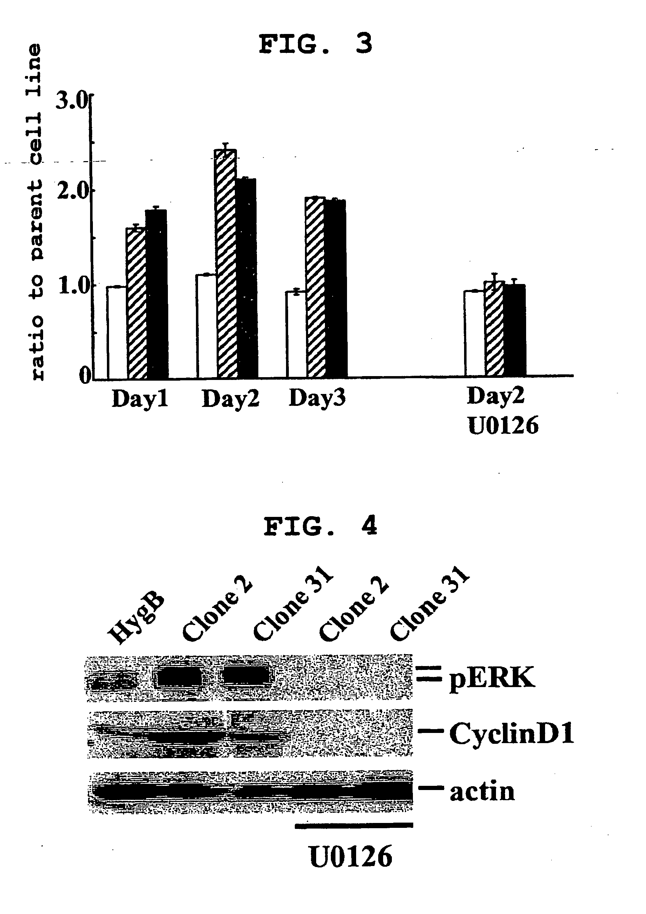

Cell growth for 36 hours under serum-free conditions was examined. Although there was no significant difference without stimulation (FIG. 2), cell growth increased by 1.5 fold to 2 fold for a PCA-1-overexpressing cell line (DU145/PCA-1) compared with a control (DU145/Neo) with stimulation with EGF (10 ng/ml); this effect was inhibited (cancelled) by a pretreatment with an inhibitor of extracellular stress-regulated kinase (ERK) (U0126) (FIG. 3).

In DU145/PCA-1, EGF-induced ERK activation and the expression of the target gene thereof, cyclin D1, increased significantly compared with the control (DU145/Neo) (FIG. 4). ERK activation was determined by searching for the expression of phospho-ERK by Western blotting.

### 2) Inhibition of FLIP ubiquitination and promotion of FLIP-Raf-1 binding by PCA-1

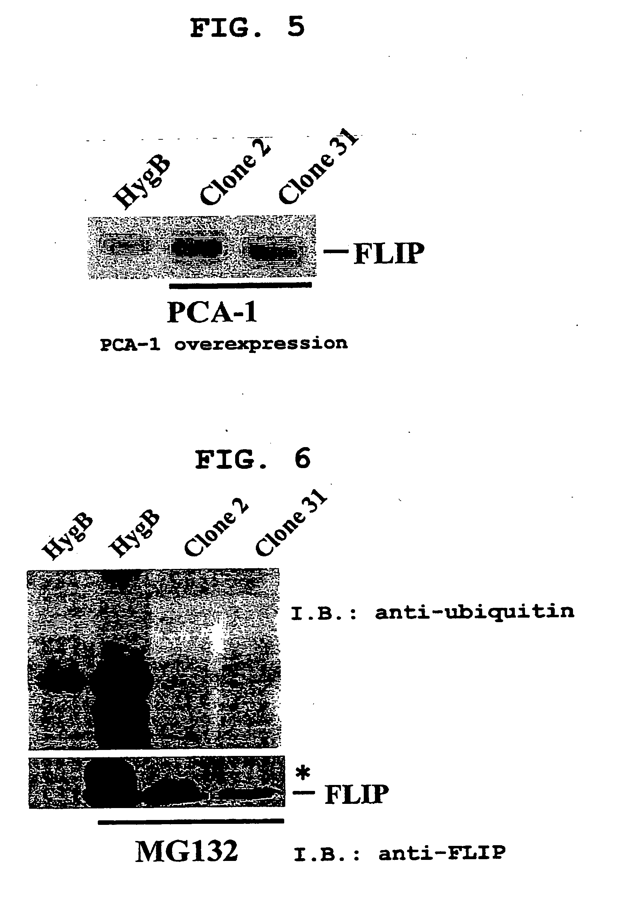

In DU145/PCA-1, the expression of FLIP protein was increased significantly compared with the control (DU145/Neo) (by Western blotting) (FIG. 5); however, as far as determined by RT-PCR, no a significant difference was observed in mRNA content. Hence, the role of PCA-1 in the ubiquitination of FLIP was analyzed. After MG132 treatment, the ubiquitination of FLIP was examined; in the control (DU145/Neo), the ubiquitination of FLIP was confirmed, but in DU145/PCA-1, the ubiquitination of FLIP was inhibited (FIG. 6).

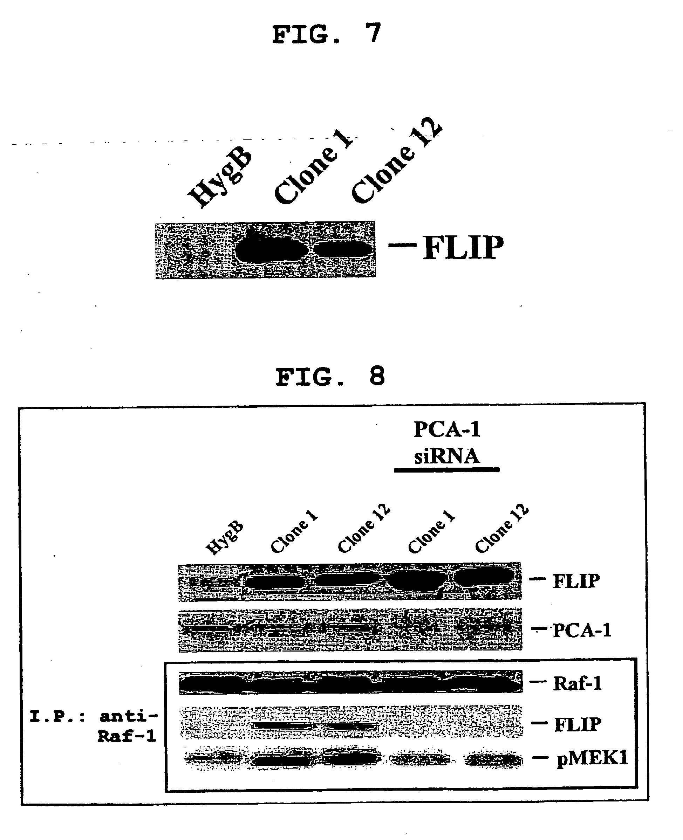

Interestingly, in DU145 wherein FLIP only was forcibly expressed (DU145/FLIP), an FLIP-Raf-1 interaction was observed, but this phenomena was inhibited (cancelled) by knocking down endogenous PCA-1 by transient forced expression of PCA-1 siRNA (FIG. 8).

From these findings, it was thought that PCA-1 not only inhibits the ubiquitination of FLIP to thereby increase the amount of FLIP protein expressed, but also influences the mutual binding (interaction) of FLIP and Raf-1 to intensify the cell growth signals from Raf-1 and below.

### 3) Role of PCA-1 in paclitaxel-induced apoptosis

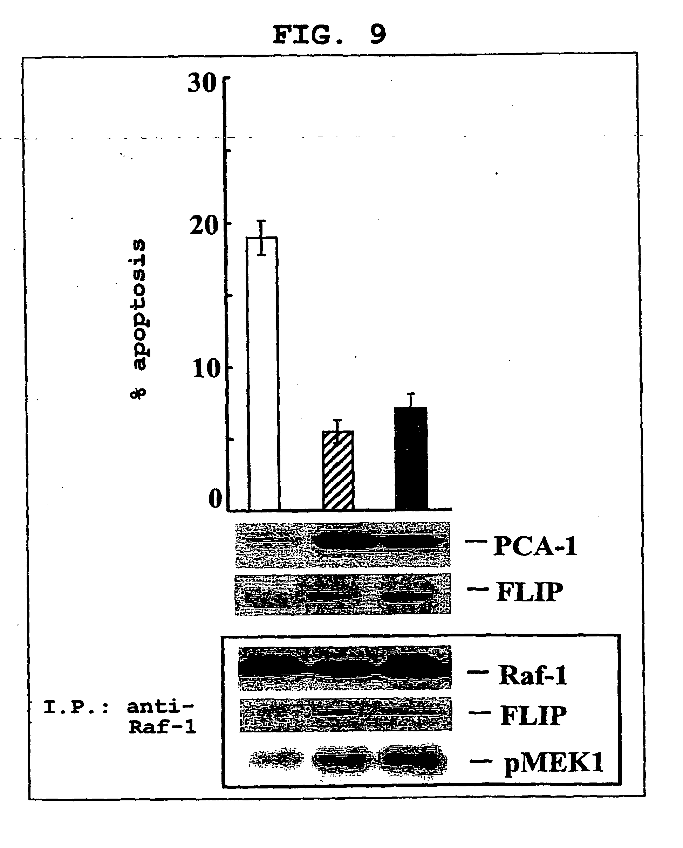

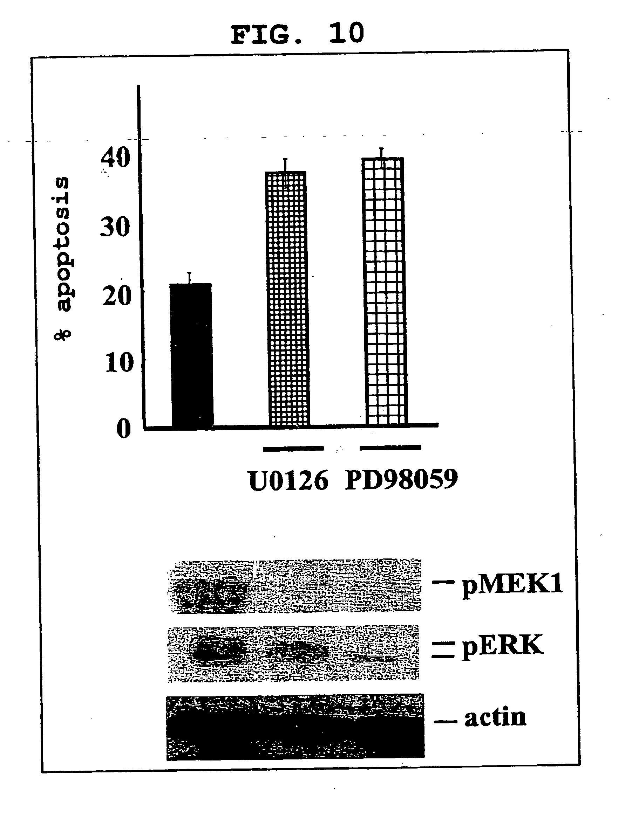

A control cell line (DU145/Neo) and DU145/PCA-1 were stimulated with paclitaxel (100 nM); 48 hours later, apoptosis was quantified using flowcytometry. As a result, in DU145/PCA-1, paclitaxel-induced apoptosis was significantly inhibited compared with the control strain (DU145/Neo) (FIG. 9). It was also found that in DU145/PCA-1, ERK activation by paclitaxel was promoted compared with the control (DU145/Neo), and that when an ERK inhibitor (U0126) was used, the paclitaxel-induced apoptosis inhibitory effect of PCA-1 was cancelled (FIG. 10).

From these findings, it was thought that PCA-1 inhibits apoptosis induction by promoting ERK activation by paclitaxel.

### 4) Role of PCA-1 in apoptosis induced by tumor necrosis factor-related apoptosis-inducing ligand (TRAIL)

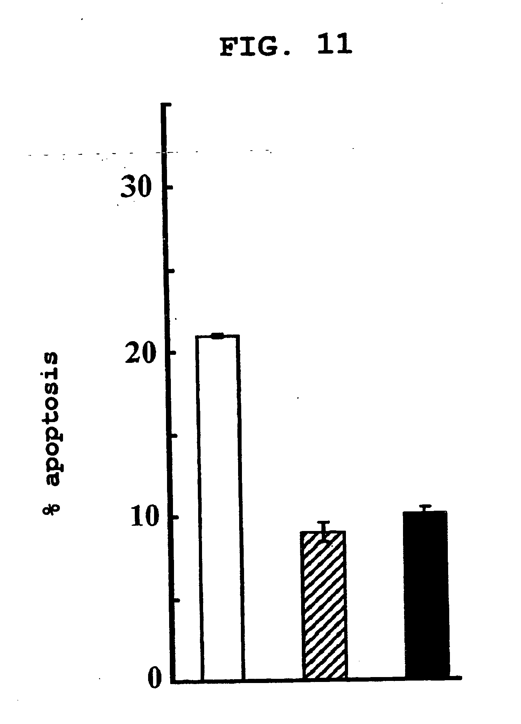

TRAIL is drawing attention as a new-generation anticancer agent for prostate cancer, and it has been reported that FLIP acts suppressively on the mechanism therefor. Hence, the effects of PCA-1 on TRAIL-induced apoptosis were analyzed. DU145/PCA-1 and control (DU145/Neo) were stimulated with TRAIL 50 ng/ml; 24 hours later, apoptosis induction was analyzed by flowcytometry; apoptosis induction was significantly inhibited in DU145/PCA-1 compared with the control (FIG. 11).

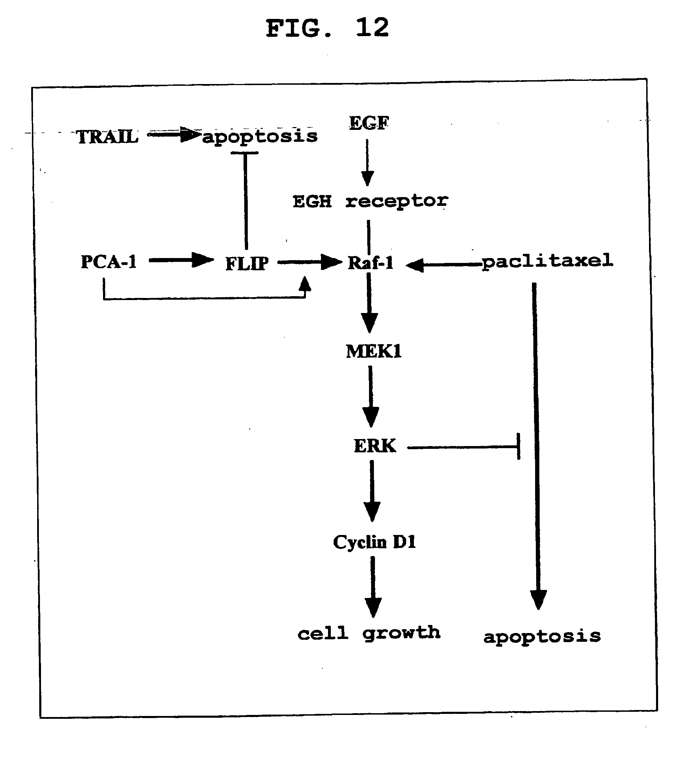

A schematic diagram showing the effects of PCA-1 on cell growth and apoptosis, suggested from the results shown above, is shown in FIG. 12.

### Example 2

### (Materials and methods)

### 1) Cell line

The human prostate cancer cell line DU145 (purchased from ATCC) was cultured in RPMI1640 medium (in the presence of 10% fetal bovine serum) and used.

### 2) Knockdown of PCA-1 with siRNA

DU145 was transiently transfected with siRNAs against PCA-1 (gaaagaagcugacuggauauu (SEQ ID NO:4), gcacauuugagaugagaaauu (SEQ ID NO:5), gagagaagcuucacugaaauu (SEQ ID NO:6)) using DharmaFECT 1 (GE Healthcare Bioscience.). Expression suppression and knockdown effects were confirmed by real-time PCR and PCA-1 quantitative ELISA method. Control siRNA (auccgcgcgauaguacgua (SEQ ID NO:7)) was purchased from GE Healthcare Bioscience.

### 3) Real-time PCR of PCA-1

Real-time PCR was performed using the SYBE ExScript RT-PCR kit (Takara Bio Inc.). The PCA-1, GAPDH-specific primers, and PCR conditions used are shown below.

**Primer base sequences**

| | |
|---|---|
| hABH3 | F; TACCACTGCTAAGAGCCATCTCC (SEQ ID NO:8) |
| | R; ACCTGCTGAGGTTCTTTGAACAC (SEQ ID NO:9) |
| GAPDH | F; CCATCACCATCTTCCAGGAG (SEQ ID NO:10) |
| | R; AATGAGCCCAGCCTTCTCC (SEQ ID NO:11) |

**Real-time PCR conditions**

| | | | | | | | |
|---|---|---|---|---|---|---|---|
| | 95°C | 9 min | | | 95°C | 30 sec | |
| | ↓ | | | | ↓ | | |
| | 95°C | 30 sec | | | 95°C | 15 sec | |
| PCA-1 | 52°C | 30 sec | × 40 | GAPDH | 67°C | 30 sec | × 45 |
| | 72°C | 15 sec | | | 72°C | 15 sec | |
| | ↓ | | | | ↓ | | |
| | 57°C | 15 sec | | | 72°C | 15 sec | |

### 4) Enzyme-linked immunosorbent assay (ELISA) of PCA-1

A trapping antibody (rabbit anti-PCA-1 antibody) was added to a 96-well plate (1 µg/well), and the plate was placed in a wet box and incubated at 4°C overnight. After the plate was washed with a PBS containing 1% Tween 20, a blocking agent was added, and the plate was allowed to stand at room temperature for 1.5 hours. After plate washing, a detection antibody (mouse anti-PCA-1 antibody clone 2-5A, 0.25 µg/well) was added, and the plate was allowed to stand at room temperature for 1.5 hours. After plate washing, an assay sample (100 µl/well) was added, and the plate was allowed to stand at room temperature for 1.5 hours. After plate washing, a tertiary antibody (HRP-labeled goat anti-mouse IgG antibody, 2 ng/well) was added, and the plate was allowed to stand at room temperature for 2 hours. After final plate washing, a color developer was added (50 µl/well); 30 minutes later, absorbance was measured at 415 nm.

### 5) WST-1 method

Two days after DU145 was transfected with siRNA, the cells were harvested, and 100 µl of the cells were sown to a 96-well plate at 1 × 10⁴/ml. Thereafter, 10 µl of a 1:9 mixture of a 1-methoxy PMS (1-methoxy-5-methylphenazinium. methylsulfate) solution and a WST-1 (2-(4-Iodophenyl)-3-(4-nitrophenyl)-5-(2,4-disulfophenyl)-2H-tetrazolium, monosodium salt) solution was added to each well every 24 hours; 1 hour later, absorbance was measured at 450 nm. As the control wavelength, 630 nm was used. The day of the start of transfection is designated as day 0.

### 6) Apoptosis analysis

After DU145 was transfected with PCA-1 siRNA, the cells were harvested and fixed with ethanol. Thereafter, the cells were stained with propidium iodide, and the ratio of apoptosis cells was analyzed using a flowcytometer.

### (Results)

### 1) Knockdown with PCA-1 siRNA

Three days after transfection with the PCA-1 siRNAs (day 3), RNA was prepared, and real-time PCR was performed. As a result, in all transfectants with the three kinds of PCA-1 siRNAs, the PCA-1 mRNA level was not more than about 20% compared with the control siRNA transfectant. When quantified at the protein level using a PCA-1 ELISA system, in the PCA-1 siRNA transfectants, the level was below the limit of detection (20 pg). This result shows that effective knockdown was achieved with the PCA-1 siRNAs used.

### 2) Induction of apoptosis by knocking-down of PCA-1

The growth of DU145 wherein PCA-1 had been knocked down was analyzed by WST-1. In the control siRNA transfected cells, remarkable growth was observed over time. Meanwhile, in the cells wherein PCA-1 had been knocked down, almost no growth was observed until day 6. The table below shows the absorbance on each measurement day relative to the absorbance on day 3 written as 1.

| Transfection | Day 3 | Day 4 | Day 5 | Day 6 | Day 7 | Day 8 |
|---|---|---|---|---|---|---|
| Control siRNA | 1 | 1.99 | 3.98 | 7.25 | 11.91 | 12.90 |
| PCA-1 siRNA | 1 | 1.14 | 1.42 | 1.35 | 1.95 | 2.45 |

### 3) Induction of apoptosis by knocking-down of PCA-1

Apoptosis of DU145 by knocking-down of PCA-1 was analyzed. On Day 2, in both the control siRNA transfectant and PCA-1 siRNA transfectant, almost no apoptosis cells were observed. However, in the cells wherein PCA-1 had been knocked down, the ratio of apoptosis cells increased remarkably over time.

| Transfection | Day 2 | Day 3 | Day 4 | Day 5 |
|---|---|---|---|---|
| Control siRNA | 0.98 % | 1.96 % | 5.00 % | 1.88 % |
| PCA-1 siRNA | 1.18 % | 10.63 % | 22.19 % | 20.99 % |

### Industrial Applicability

Because a compound that suppresses the expression or function of PCA-1, which is an active ingredient of the agent of the present invention, cancels the apoptosis inhibitory action and cell growth enhancing action of PCA-1, and reduces the anticancer-agent resistance and cell growth of cancer cells, it is useful as an apoptosis promoting agent, a cell growth inhibitor, and a prophylactic/therapeutic agent for cancer. By selecting a compound capable of suppressing the expression or function of PCA-1, it is possible to screen for a compound for promoting apoptosis, a compound for inhibiting cell growth, and a compound for preventing/ treating cancer, having a novel mechanism of action.

## Claims

1. A compound for use in preventing or treating cancer by promoting apoptosis which is induced by FASL, TRAIL or TNF-α, wherein the compound is (i) or (ii) below:
(i) a compound that suppresses the expression of PCA-1 that is a nucleic acid having a nucleotide sequence or a portion thereof complementary to the nucleotide sequence that encodes the PCA-1 polypeptide;
(ii) a compound that suppresses the function of PCA-1 that is an antibody that specifically recognizes the PCA-1 polypeptide or a nucleic acid having the nucleotide sequence that encodes the same.

2. A compound for use in preventing or treating cancer by promoting apoptosis which is induced by paclitaxel, wherein the compound is (i) or (ii) below:
(i) a compound that suppresses the expression of PCA-1 that is a nucleic acid having a nucleotide sequence or a portion thereof complementary to the nucleotide sequence that encodes the PCA-1 polypeptide;
(ii) a compound that suppresses the function of PCA-1that is an antibody that specifically recognizes the PCA-1 polypeptide or a nucleic acid having the nucleotide sequence that encodes the same.

3. A compound for use in the prophylactic/therapeutic treatment of a prostate cancer, wherein the compound is (i) or (ii) below;
(i) a compound that suppresses the expression of PCA-1 that is a nucleic acid having a nucleotide sequence or a portion thereof complementary to the nucleotide sequence that encodes the PCA-1 polypeptide;
(ii) a compound that suppresses the function of PCA-1 that is an antibody that specifically recognizes the PCA-1polypeptide or a nucleic acid having the nucleotide sequence that encodes the same.

4. An *in vitro* screening method for a compound for promoting the ubiquitination of FLIP, comprising the following steps:
(1) a step for treating PCA-1 expressing cells with a test compound in the presence of proteasome inhibitor;
(2) a step for evaluating the ubiquitination of FLIP in said cells.

5. An *in vitro* screening method for a compound for preventing or treating prostate cancer, comprising the following steps:
(1) a step for treating PCA-1-expressing cells with paclitaxel in the presence of a test compound;
(2) a step for evaluating the degree of the apoptosis of said cells.

6. The method described in claim 4 wherein a compound for preventing/treating cancer is identified.

7. The method of claim 6, wherein the cancer is prostate cancer.

## Patentansprüche

1. Verbindung zur Verwendung bei der Prävention oder Behandlung von Krebs durch Förderung der Apoptose, die durch FASL, TRAIL oder TNF-α induziert wird, wobei es sich bei der Verbindung um die folgende (i) oder (ii) handelt:
(i) eine Verbindung, die die Expression von PCA-1 unterdrückt und bei der es sich um eine Nucleinsäure handelt, die eine Nucleotidsequenz, die zu der Nucleotidsequenz, die das PCA-1-Polypeptid codiert, komplementär ist, oder einen Teil davon aufweist;
(ii) eine Verbindung, die die Funktion von PCA-1 unterdrückt und bei der es sich um einen Antikörper handelt, der das PCA-1-Polypeptid oder eine Nucleinsäure, die die dasselbe codierende Nucleotidsequenz aufweist, spezifisch erkennt.

2. Verbindung zur Verwendung bei der Prävention oder Behandlung von Krebs durch Förderung der Apoptose, die durch Paclitaxel induziert wird, wobei es sich bei der Verbindung um die folgende (i) oder (ii) handelt:
(i) eine Verbindung, die die Expression von PCA-1 unterdrückt und bei der es sich um eine Nucleinsäure handelt, die eine Nucleotidsequenz, die zu der Nucleotidsequenz, die das PCA-1-Polypeptid codiert, komplementär ist, oder einen Teil davon aufweist;
(ii) eine Verbindung, die die Funktion von PCA-1 unterdrückt und bei der es sich um einen Antikörper handelt, der das PCA-1-Polypeptid oder eine Nucleinsäure, die die dasselbe codierende Nucleotidsequenz aufweist, spezifisch erkennt.

3. Verbindung zur Verwendung bei der prophylaktischen/therapeutischen Behandlung von Prostatakrebs, wobei es sich bei der Verbindung um die folgende (i) oder (ii) handelt:
(i) eine Verbindung, die die Expression von PCA-1 unterdrückt und bei der es sich um eine Nucleinsäure handelt, die eine Nucleotidsequenz, die zu der Nucleotidsequenz, die das PCA-1-Polypeptid codiert, komplementär ist, oder einen Teil davon aufweist;
(ii) eine Verbindung, die die Funktion von PCA-1 unterdrückt und bei der es sich um einen Antikörper handelt, der das PCA-1-Polypeptid oder eine Nucleinsäure, die die dasselbe codierende Nucleotidsequenz aufweist, spezifisch erkennt.

4. In-Vitro-Screeningverfahren für eine Verbindung zur Förderung der Ubiquitinierung von FLIP, umfassend die folgenden Schritte:
(1) einen Schritt zur Behandlung von PCA-1-exprimierenden Zellen mit einer Testverbindung in Gegenwart eines Proteasom-Inhibitors;
(2) einen Schritt zur Bewertung der Ubiquitinierung von FLIP in diesen Zellen.

5. In-Vitro-Screeningverfahren für eine Verbindung zur Prävention oder Behandlung von Prostatakrebs, umfassend die folgenden Schritte:
(1) einen Schritt zur Behandlung von PCA-1-exprimierenden Zellen mit Paclitaxel in Gegenwart einer Testverbindung;
(2) einen Schritt zur Bewertung des Grades der Apoptose bei diesen Zellen.

6. Verfahren gemäß Anspruch 4, wobei eine Verbindung zur Prävention/Be-handlung von Krebs identifiziert wird.

7. Verfahren gemäß Anspruch 6, wobei es sich bei dem Krebs um Prostatakrebs handelt.

## Revendications

1. Composé pour son utilisation dans la prévention ou le traitement du cancer, en favorisant l'apoptose qui est induite par FASL, TRAIL ou TNF-α, le composé étant (i) ou (ii) ci-dessous :
(i) un composé qui supprime l'expression de PCA-1 qui est un acide nucléique ayant une séquence nucléotidique ou une partie de celle-ci complémentaire à la séquence nucléotidique qui code pour le polypeptide PCA-1 ;
(ii) un composé qui supprime la fonction de PCA-1 qui est un anticorps qui reconnaît spécifiquement le polypeptide PCA-1 ou un acide nucléique ayant la séquence nucléotidique qui code pour celui-ci.

2. Composé pour son utilisation dans la prévention ou le traitement du cancer en favorisant l'apoptose qui est induite par le paclitaxel, le composé étant (i) ou (ii) ci-dessous :
(i) un composé qui supprime l'expression de PCA-1 qui est un acide nucléique ayant une séquence nucléotidique ou une partie de celle-ci complémentaire à la séquence nucléotidique qui code pour le polypeptide PCA-1 ;
(ii) un composé qui supprime la fonction de PCA-1 qui est un anticorps qui reconnaît spécifiquement le polypeptide PCA-1 ou un acide nucléique ayant la séquence nucléotidique qui code pour celui-ci.

3. Composé pour son utilisation dans le traitement prophylactique/thérapeutique d'un cancer de la prostate, le composé étant (i) ou (ii) ci-dessous ;
(i) un composé qui supprime l'expression de PCA-1 qui est un acide nucléique ayant une séquence nucléotidique ou une partie de celle-ci complémentaire à la séquence nucléotidique qui code pour le polypeptide PCA-1 ;
(ii) un composé qui supprime la fonction de PCA-1 qui est un anticorps qui reconnaît spécifiquement le polypeptide PCA-1 ou un acide nucléique ayant la séquence nucléotidique qui code pour celui-ci.

4. Méthode de criblage *in vitro* d'un composé pour favoriser l'ubiquitination de FLIP, comprenant les étapes suivantes ;
(1) une étape pour traiter des cellules exprimant PCA-1 avec un composé de test en présence d'un inhibiteur de protéasome ;
(2) une étape d'évaluation de l'ubiquitination de FLIP dans lesdites cellules.

5. Méthode de criblage *in vitro* d'un composé pour la prévention ou le traitement du cancer de la prostate, comprenant les étapes suivantes ;
(1) une étape pour traiter des cellules exprimant PCA-1 avec du paclitaxel en présence d'un composé de test ;
(2) une étape d'évaluation du degré d'apoptose desdites cellules.

6. Méthode décrite dans la revendication 4, dans laquelle un composé pour prévenir/traiter le cancer est identifié.

7. Méthode selon la revendication 6, dans laquelle le cancer est le cancer de la prostate.
